(19) Europäisches Patentamt
European Patent Office
Office européen des brevets

(11) Publication number: **0 476 944 B1**

# (12) EUROPEAN PATENT SPECIFICATION

(45) Date of publication of patent specification: **15.11.95**

(51) Int. Cl.⁶: **C07D 233/64**, A61K 31/415

(21) Application number: **91308407.5**

(22) Date of filing: **13.09.91**

The file contains technical information submitted after the application was filed and not included in this specification

## (54) Aromatase inhibiting 4(5)-imidazoles.

(30) Priority: **21.09.90 GB 9020629**

(43) Date of publication of application:
**25.03.92 Bulletin 92/13**

(45) Publication of the grant of the patent:
**15.11.95 Bulletin 95/46**

(84) Designated Contracting States:
**AT BE CH DE DK ES FR GB GR IT LI LU NL SE**

(56) References cited:
**EP-A- 0 165 779**
**EP-A- 0 194 984**
**EP-A- 0 311 447**
**EP-A- 0 390 558**

(73) Proprietor: **ORION-YHTYMÄ OY**
**Orionintie 1,**
**PL 65**
**SF-02101 Espoo (FI)**

(72) Inventor: **Karjalainen, Arto Johannes**
**Myllyojantie 13 H 37**
**SF-90650 Oulu (FI)**
Inventor: **Kalapudas, Arja Marketta**
**Notkolantie 13**
**SF-90240 Oulu (FI)**
Inventor: **Pelkonen, Reino Olavi**
**Hamalantie 3 C**
**SF-90800 Oulu (FI)**
Inventor: **Sodervall, Marja-Liisa**
**Annantie 9-13 C 7**
**SF-90650 Oulu (FI)**
Inventor: **Lahde, Matti Antero**
**Itainen Pitkakatu 5 B 35**
**SF-20520 Turku (FI)**
Inventor: **Lammintausta, Risto Arvo Sakari**
**Meltoistentie**
**SF-20900 Turku (FI)**

(74) Representative: **Sexton, Jane Helen et al**
**J.A. KEMP & CO.**
**14 South Square**
**Gray's Inn**
**London WC1R 5LX (GB)**

EP 0 476 944 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to substituted imidazole derivatives and their non-toxic, pharmaceutically acceptable acid addition salts, and their preparation, to pharmaceutical compositions containing the same and their use.

EP-A-0 194 984 discloses imidazole derivatives that have $\alpha_2$-blocking and/or anti-convulsive activities which are said to be utilisable as therapeutic agents. EP-A-0 311 447 discloses imidazole derivatives which are said to exhibit pharmacological properties, especially aromatase inhibiting effects and are said to be useful in the treatment of estrogen dependent diseases, e.g. breast cancer. EP-A-0 165 779 discloses aromatase inhibiting 4(5)-substituted imidazole derivatives, a process for preparing them, their use to inhibit aromatase or to treat or prevent estrogen dependent disease, and pharmaceutical formulations comprising the derivatives as active ingredient. EP-A-0 390 558 which was published on 3 October 1990 discloses imidazole derivatives which are said to exhibit aromatase and desmolase inhibiting properties and it suggests that very selective aromatase inhibiting compounds are valuable in the treatment of estrogen dependent diseases, e.g. breast cancer.

The imidazole derivatives of the present invention have the general formula (I):

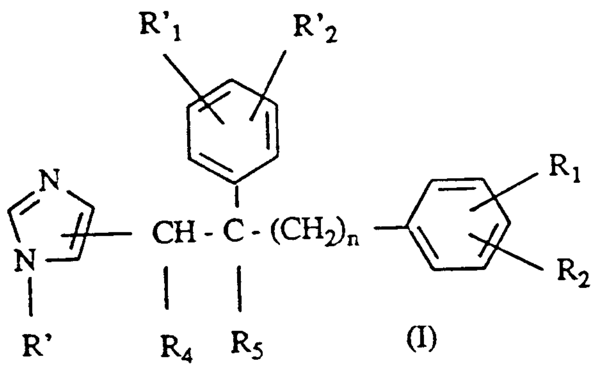

(I)

wherein $R_1$, $R_2$, $R'_1$ and $R'_2$, which can be the same or different, are H, $CH_3$, $C_2H_5$, $C_3H_7$, $OCH_3$, $NO_2$, $NH_2$, CN, $CF_3$, $CHF_2$, $CH_2F$ or halogen; R' is H or

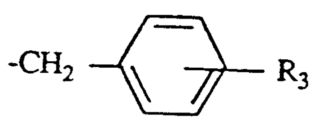

,

where $R_3$ is H, $CH_3$ or halogen; $R_4$ is H or OH and $R_5$ is H or OH or $R_4$ and $R_5$ together form a bond and n is 1 to 4.

The non-toxic pharmaceutically acceptable acid addition salts of these compounds are also within the scope of the invention.

The compounds of formula (I) form acid addition salts with both organic and inorganic acids. They can thus form many pharmaceutically usable acid addition salts, as for instance, chlorides, bromides, sulfates, nitrates, phosphates, sulfonates, formates, tartrates, maleates, citrates, benzoates, salicylates, ascorbates and the like.

Preferred values for $R_1$, $R_2$, $R'_1$ and $R'_2$ are H, CN or halogen, especially H, CN or F. The phenyl groups may be substituted in the ortho, meta or para position. If a phenyl group is mono-substituted, it is preferably substituted in the para-position.

Preferably R' is H; preferably $R_4$ and $R_5$ are each H.

The invention includes within its scope pharmaceutical compositions comprising at least one compound of formula (I) or a non-toxic pharmaceutically acceptable salt thereof, and a compatible pharmaceutically acceptable carrier therefor. The compounds of the present invention have been found, depending on the substituents R', $R_1$, $R_2$, $R'_1$ and $R'_2$, to possess varying degrees of aromatase and desmolase inhibiting properties. Among them there are very selective enzyme aromatase inhibiting compounds which are valuable in the treatment of estrogen dependent diseases, e.g. breast cancer or benign prostatic hyperplasia (BPH).

The present invention also provides compounds of formula (I) or non-toxic pharmaceutically acceptable acid addition salts thereof for use in a method of medical treatment. The present invention further provides the use of compound of formula (I) or non-toxic pharmaceutically acceptable acid addition salts thereof in the manufacture of a medicament for the inhibition of aromatase.

Compounds of formula (I) can be prepared by McMurry reaction which comprises a reductive coupling of a diphenylketone (II)

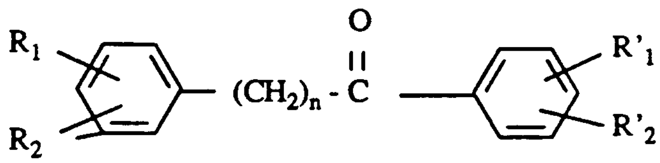

(II)

wherein $R_1$, $R_2$, $R'_1$ and $R'_2$, which can be the same or different, are H, $CH_3$, $C_2H_5$, $C_3H_7$, $OCH_3$, $NH_2$, CN, $CF_3$, $CHF_2$, $CH_2F$ or halogen and n is 1-4, and an 4(5)-imidazole aldehyde (III)

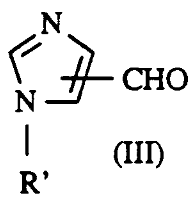

(III)

wherein R' is as defined before, in an appropriate solvent, e.g. tetrahydrofuran or dimethoxyethane, in the presence of a low valent titanium reagent in an inert atmosphere, e.g. in nitrogen or argon, to give the compounds of formula (I) where $R_4$ and $R_5$ together form a bond (IV)

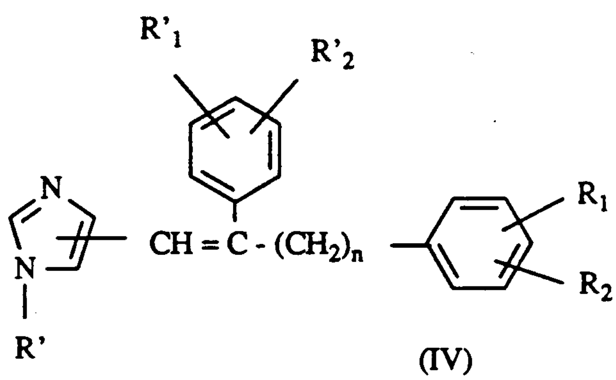

(IV)

The unsaturated compounds (IV) are isolated and after that hydrogenated. Alternatively they can be hydrogenated directly in an acidic medium without previous isolation. The hydrogenation is conveniently carried out at room temperature with good stirring in alcohol, e.g. ethanol, in the presence of a catalyst in a hydrogen atmosphere. Suitable catalysts are for example platinium oxide, palladium-on-carbon or Raney-nickel.

The reaction scheme for this step can be illustrated as follows:

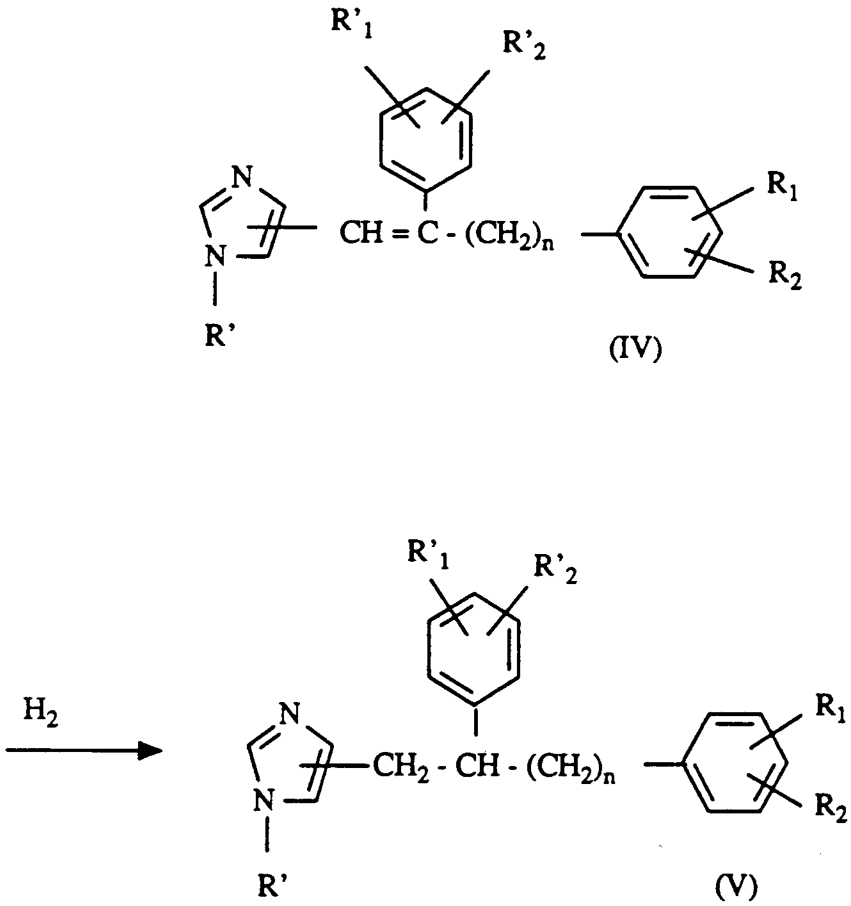


(IV)

(V)

If R' is a substituted or unsubstituted benzyl, this group may be removed by hydrogenation as well. In this case the hydrogenation is performed in an acidic medium such as hydrochloric acid-ethanol mixture.

The reaction scheme of this hydrogenation which leads to compounds of formula (I) wherein R', $R_4$ and $R_5$ each are hydrogen can be illustrated as follows:

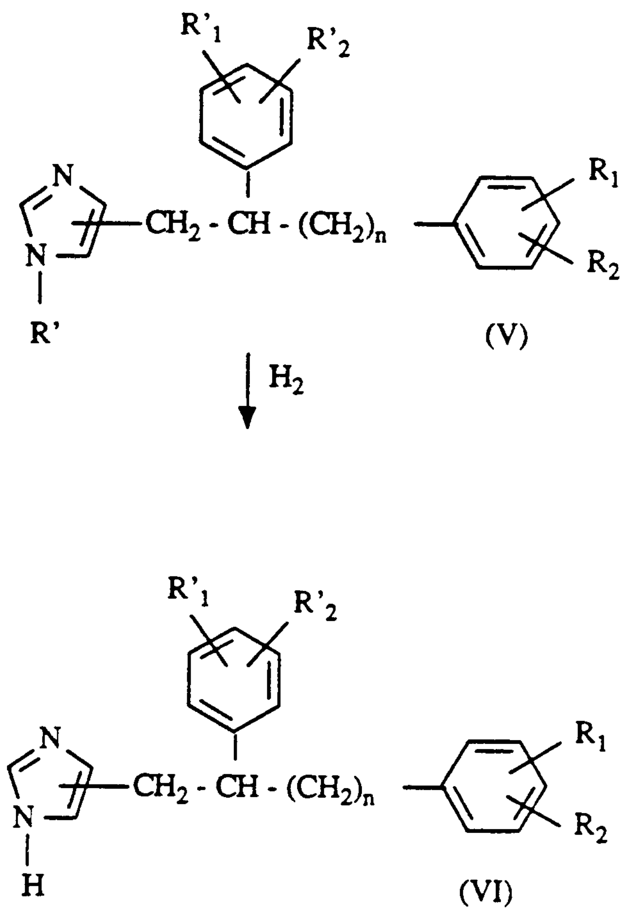


(V)

(VI)

Another method to remove the benzylic R' group is a hydrogen transfer reaction in which the starting compound (V) is refluxed with ammonium formate and 10 % Pd/C in an appropriate lower alcohol, such as methanol or ethanol, or its aqueous solution. The compounds (VI) can also be prepared from the compounds (IV) by hydrogen transfer reaction with ammonium formate or by hydrogenating both the double bond and the protecting benzyl group at the same time. If one or more of the substituents $R_1$, $R_2$, $R'_1$ and $R'_2$ is CN, it must be protected during the hydrogen transfer reaction.

The ketone of formula (II) can be prepared for example from an appropriately substituted acetophenone and benzaldehyde by condensation and hydrogenation.

Another method of preparing compounds of formula (I) is a reaction which comprises reacting a ketone of formula (VII)

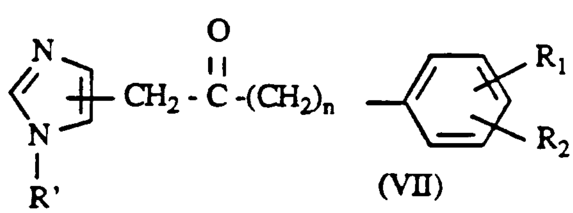

(VII)

wherein R' and n are as defined before and $R_1$ and $R_2$, which can be the same or different, are H, $CH_3$, $C_2H_5$, $C_3H_7$, $OCH_3$, $NO_2$, $CF_3$, $CHF_2$, $CH_2F$ or halogen, with an appropriate halide derivative of the formula (VIII)

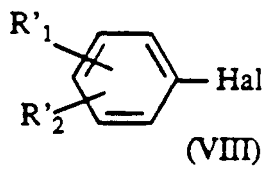

(VIII)

wherein $R'_1$ and $R'_2$, which can be the same or different, are H, $CH_3$, $C_2H_5$, $C_3H_7$, $OCH_3$, $NO_2$, $CF_3$, $CHF_2$, $CH_2F$ or halogen, in the presence of an alkyl lithium, such as n-butyl lithium, or magnesium in an appropriate solvent, such as tetrahydrofuran, to give compounds of formula (IX)

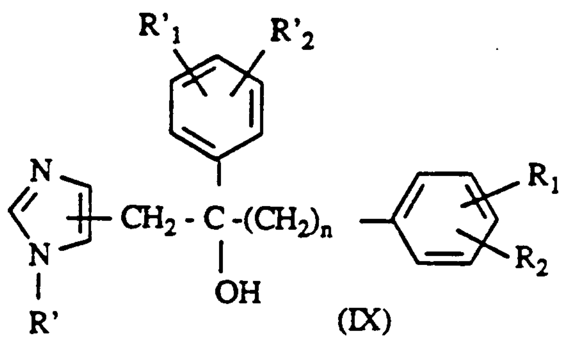

(IX)

wherein R', $R_1$, $R_2$, $R'_1$, $R'_2$ and n are as defined for formulae (VII) and (VIII). Compounds of formula (IX) are further dehydrated to form compounds of formula (I) where $R_4$ and $R_5$ together form a bond (IV). Water is eliminated by usual methods, i.e. by heating with dry potassium hydrogen sulfate. The unsaturated compounds (IV) are hydrogenated by the methods described before. The benzylic R' can also be removed from the compounds of formula (IX) by a hydrogen transfer reaction to give compounds of formula (X)

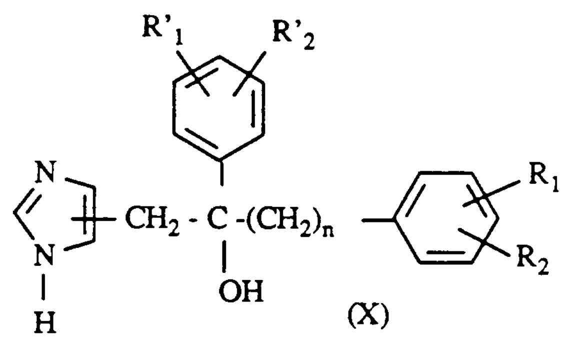

(X)

wherein $R_1$, $R_2$, $R'_1$ and $R'_2$ are as defined for formula (IX).

Compounds of formula (VII) can be prepared e.g. from an 4(5)-imidazole acetaldehyde and an appropriate arylalkylhalide and then oxidizing.

Compounds of formula (I) wherein one or more of the substituents are CN can also be prepared by the method described above but then the CN group(s) in the formulae (VII) and (VIII) must be protected by a tert-butylaminocarbonyl group or an oxazoline group and R' is a protecting benzyl group. $NO_2$ cannot be one of the substituents. Compounds of formula (IX) wherein one or more of the substituents are a tert-butylaminocarbonyl group or an oxazoline group (IX') are dehydrated and hydrogenated by the methods described before and further refluxed in $SOCl_2$ to give compounds of formula (VI) wherein one or more of the substituents $R_1$, $R_2$, $R'_1$ and $R'_2$ are CN.

Compounds of formula (I) wherein one or more of the substituents $R_1$, $R_2$, $R'_1$ and $R'_2$ are CN can also be prepared by refluxing compounds of formula (IX) wherein one or more of the substituents are a tert-butylaminocarbonyl group or an oxazoline group (IX') with e.g. $SOCl_2$, $POCl_3$ or $PCl_5$ optionally in an appropriate solvent, such as acetonitrile, to give compounds of formula (I) wherein R' is a benzyl group, $R_4$ and $R_5$ form together a bond and one or more of the substituents $R_1$, $R_2$, $R'_1$ and $R'_2$ are CN. The

substituents that are not CN are H, $CH_3$, $C_2H_5$, $C_3H_7$, $OCH_3$, $CF_3$, $CHF_2$, $CH_2F$ or halogen. The unsaturated compounds are hydrogenated by the methods described before.

Compounds of formula (X) wherein one or more of the substituents are a tert-butylaminocarbonyl or an oxazoline group (X') may be dehydrated by e.g. $SOCl_2$, $POCl_3$ or $PCl_5$ as described before to form compounds of formula (XI)

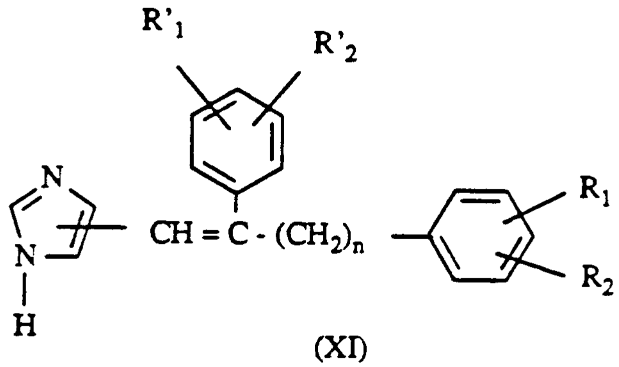

(XI)

wherein one or more of the substituents $R_1$, $R_2$, $R'_1$ and $R'_2$ are CN and the substituents that are not CN are H, $CH_3$, $C_2H_5$, $C_3H_7$, $OCH_3$, $CF_3$, $CHF_2$, $CH_2F$ or halogen.

Another method to give compounds of formula (I) wherein the substituents are as defined for formula (XI) is refluxing compounds of formula (IX') or (X'), in an appropriate solvent, such as dichloromethane, in the presence of $SOCl_2$ to give compounds of formula (IV) wherein R' is H or a protecting group and the substituents $R_1$, $R_2$, $R'_1$ and $R'_2$ are as defined for formula (X'). The unsaturated compounds are further reacted by a hydrogen transfer reaction or hydrogenated to give compounds of formula (VI) wherein the substituents are as defined for formula (X') which are further reacted with e.g. $SOCl_2$ to give compounds of formula (I) wherein the substituents are as defined for formula (XI).

Compounds of formula (VI) wherein the substituents are as defined for formula (X') can be prepared by a hydrogen transfer reaction from compounds of formula (IX').

The compounds of formula (I) wherein one or more of the substituents are CN can be prepared from the corresponding compounds where one or more of the substituents $R_1$, $R_2$, $R'_1$ and $R'_2$ are $NH_2$ by diazotization.

Compounds of formula (I) wherein one or more of the substituents $R_1$, $R_2$, $R'_1$ and $R'_2$ are $NH_2$ can be prepared by hydrogenating the corresponding compounds where one or more of the substituents are $NO_2$. Compounds of formula (I) wherein one or more of the substituents are $NO_2$ can be prepared by nitration.

Compounds of formula (I) where R' is a benzyl group can be prepared by benzylating the corresponding compounds where R' is hydrogen. The starting compound is first treated with a strong base such as sodium hydroxide in water or sodium hydride in an appropriate solvent, e.g. dimethyl formamide, to give the alkali metal salt of the imidazole and then in the second step adding to this benzyl halide. The reaction scheme can be illustrated as follows:

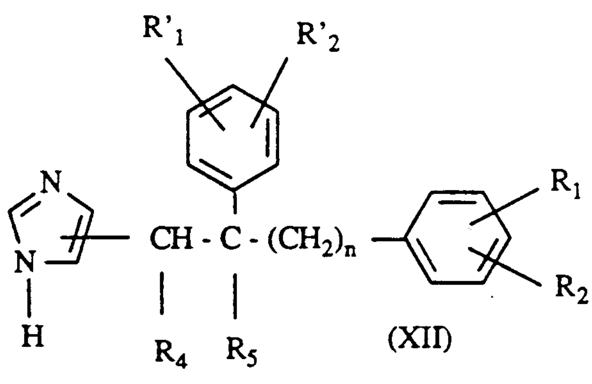

(XII)

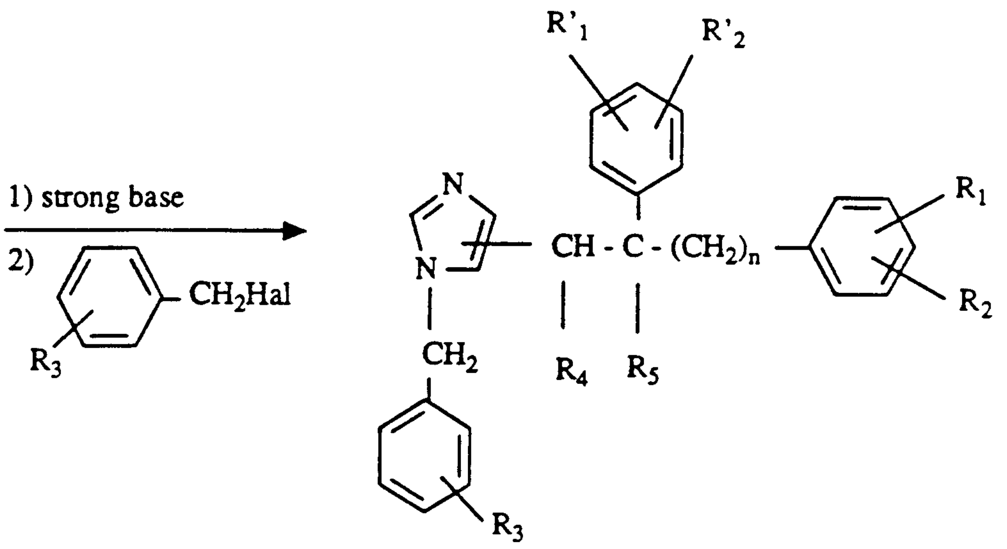


(XIII)

The free $NH_2$ substituent must be protected during the benzylation reaction.

Another method for the preparation of compounds of formula (IV) is the reaction of a diphenylhalogen compound (XIV)

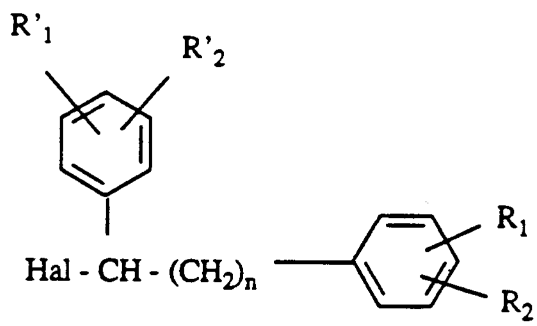

(XIV)

wherein $R_1$, $R_2$, $R'_1$ and $R'_2$, which can be the same or different, are H, $CH_3$, $C_2H_5$, $C_3H_7$, $OCH_3$, $NO_2$, $CF_3$, $CHF_2$, $CH_2F$ or halogen, n is 1 to 4 and Hal is halogen, with an 4(5)-imidazole aldehyde (III) using n-butyllithium as a reagent to give compounds of formula (XV)

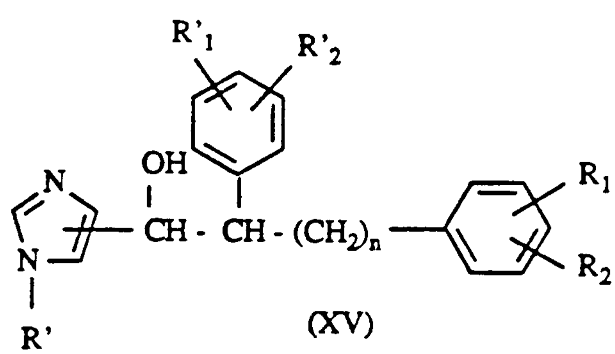

(XV)

The compounds of formula (XV) may be dehydrated to give the compounds of formula (IV) wherein the substituents $R_1$, $R_2$, $R'_1$ and $R'_2$, which can be the same or different, are H, $CH_3$, $C_2H_5$, $C_3H_7$, $OCH_3$, $NO_2$,

$CF_3$, $CHF_2$, $CH_2F$ or halogen. The compounds of formula (IV) wherein one or more of the substituents are CN may be prepared by the same method when the CN-group(s) is(are) protected.

Still another method for preparing of compounds of formula (I) is a process which comprises a Reformatskii reaction of the 4(5)-imidazole aldehyde (III) with an ester of α-bromophenylacetic acid (VXI)

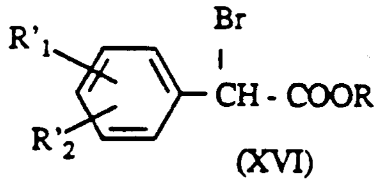

(XVI)

wherein $R'_1$ and $R'_2$, which can be the same or different, are H, $CH_3$, $C_2H_5$, $C_3H_7$, $OCH_3$, $NO_2$, $CF_3$, $CHF_2$, $CH_2F$ or halogen and R is a lower alkyl, to give an ester of β-hydroxy-α-phenylimidazolepropionic acid (XVII)

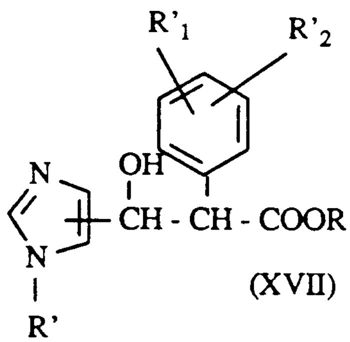

(XVII)

The compounds of formula (XVII) can be dehydrated and hydrogenated to give the saturated α-phenylimidazolepropionic acid esters (XVIII) which can be reduced to aldehydes of formula (XIX)

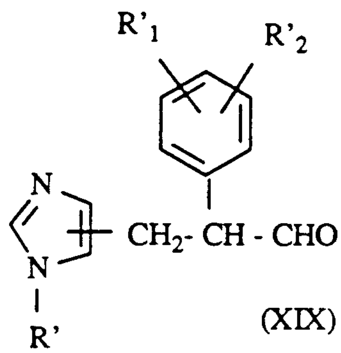

(XIX)

or converted to amides of formula (XX)

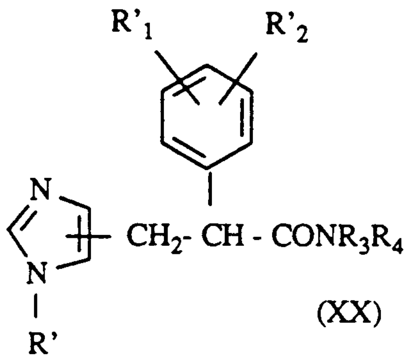

(XX)

wherein $R_3$ and $R_4$ are lower alkyl or N, $R_3$ and $R_4$ together form a ring. The reaction of the compounds of formula (XIX) or (XX) with an appropriate aryl- or arylalkyl halide (XXI)

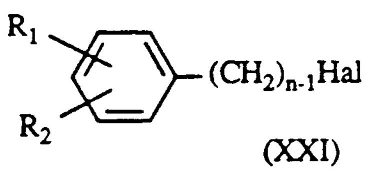

(XXI)

wherein $R_1$ and $R_2$, which can be the same or different, are H, $CH_3$, $C_2H_5$, $C_3H_7$, $OCH_3$, $NO_2$, $NH_2$, CN, $CF_3$, $CHF_2$, $CH_2F$ or halogen and n is 1 to 4, in the presence of alkyl lithium, e.g. n-butyl lithium, or magnesium give alcohols (XXII) or ketones (XXIII) respectively. The substituents $R_1$ and $R_2$ of the halide (XXI) must be protected if they are $NH_2$ or CN.

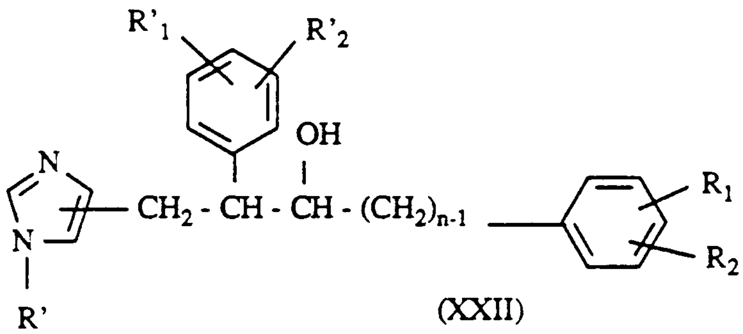

(XXII)

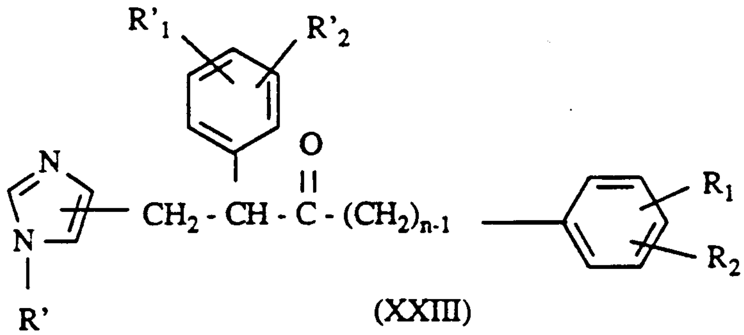

(XXIII)

The compounds of formulae (XXII) and (XXIII) can further be reduced, dehydrated and hydrogenated to give the compounds of formula (I) wherein $R_1$, $R_2$, $R'_1$ and $R'_2$, which can be the same or different, are H, $CH_3$, $C_2H_5$, $C_3H_7$, $OCH_3$, $NH_2$, CN, $CF_3$, $CHF_2$, $CH_2F$ or halogen.

The compounds of formula (I), their non-toxic, pharmaceutically acceptable acid addition salts or mixtures thereof may be administered parenterally, intravenously or orally. Typically, an effective amount of the compound is combined with a suitable pharmaceutical carrier. As used herein, the term "effective amount" encompasses those amounts which yield the desired activity without causing adverse side-effects. The precise amount employed in a particular situation is dependent upon numerous factors such as method of administration, type of mammal, condition for which the compound is administered, etc., and of course the structure of the compound.

The pharmaceutical carriers which are typically employed with the compounds of the present invention may be solid or liquid and are generally selected with the planned manner of administration in mind. Thus, for example, solid carriers include lactose, sucrose, gelatin and agar, while liquid carriers include water, syrup, peanut oil and olive oil. Other suitable carriers are well known to those skilled in the art of pharmaceutical formulations. Other combination of the compound and the carrier may be fashioned into numerous acceptable forms, such as tablets, capsules, suppositories, solutions, emulsions and powders.

The compounds of the invention are especially valuable as aromatase inhibiting agents and are therefore useful in the treatment of estrogen dependent diseases, e.g. breast cancer or benign prostatic hyperplasia (BPH).

Estrogens are essential steroids in the physiology and function of normal development of breast and sex organs in women. On the other hand estrogens are known to stimulate the growth of estrogen dependent cancers, especially breast and endometrial cancers, and they may increase the risk of development of breast cancer if given at pharmacological doses for a long time. Excessive production of estradiol may also cause other, benign disorders in hormone dependent organs. The importance of estrogens as cancer growth stimulators and/or regulators is clearly stressed by the fact that antiestrogens have reached a central position in the treatment of estrogen receptor rich breast cancers. Antiestrogens act by binding to estrogen receptors and thereby inhibiting the biological effect of estrogens. This has been achieved clinically by the unspecific steroid synthesis inhibitor aminoglutethimide. The estrogen synthesis could be blocked specifically by inhibiting the enzyme aromatase, which is the key enzyme in biochemical estrogen synthesis pathway. Aromatase inhibition is important because several breast tumors synthesize

estradiol and estrone in situ and exhibit therefore continuous growth stimulation (Alan Lipton et al., Cancer 59:770-782, 1987).

The ability of the compounds of the invention to inhibit the enzyme aromatase was shown by the in vitro assay method according to M. Pasanen (Biological Research in Pregnancy, vol. 6, No. 2, 1985, pp. 94-99). Human aromatase enzyme was used. The enzyme was prepared from human placenta, which is rich of the enzyme. Microsomal fraction (100000 x g precipitate) was prepared by centrifugation. The enzyme preparation was used without further purification. Test compounds listed in Table 1 were added together with 100000 dpm of 1,2[$^3$H]-androstene-3,17-dione and NADPH generating system. The concentrations of the test compounds were 0,001; 0,01; 0,1 and 1,0 mM. The incubation was carried out at 37 °C for 40 min. Aromatization of 1,2[$^3$H]-androstene-3,17-dione results in the production of $^3H_2O$. The tritiated water and the tritiated substrate are easily separated by a Sep-Pak$^R$ minicolumn, which absorbs the steroid but allows free water elution. Radioactivity was counted by a liquid scintillation counter. Aromatase inhibition was evaluated by comparing the $^3H_2O$-radioactivity of inhibitor treated samples to controls containing no inhibitor. IC-50 values were calculated as concentrations which inhibited the enzyme activity 50 %. These concentrations are presented in Table 2.

Cholesterol side chain cleavage (SCC) activity (desmolase) was measured according to the method of Pasanen and Pelkonen (Steroids 43:517-527, 1984). Incubations were carried out in 1,5 ml Eppendorf plastic tubes, and an Eppendorf shaker, centrifuge and incubator were used as a unit. In a 300 µl incubation volume, the substrate (5 µM) was prepared according to Hanukoglu and Jefcoate (J. Chromatogr. 190:256-262, 1980), and 100000 dpm of radioactive $^3$H-4-cholesterol (the purity of the compound was checked by TLC) in 0,5 % Tween 20, 10 mM $MgCl_2$, 5 µM cyanoketone and 2 mM NADPH was added. Controls contained all the above substances but the enzyme preparation was inactivated prior to the incubation by the addition of 900 µl of methanol. The mitochondrial fraction (1 mg protein) from human placenta or bovine adrenals was used as a source of enzyme. After 30 min incubation at 37 °C, the reaction was terminated by the addition of 900 µl of methanol; 1500 dpm of marker $^{14}$C-4-pregnenolone was added to each incubate and the tubes were vigorously shaken. After 10 min equilibration, the methanol-precipitated proteins were separated by centrifugation (8000 x g for 2 min) and the supernatant was sucked into 1 ml plastic injection syringe and loaded onto the pre-equilibrated (75 % methanol) minicolumn. The column was washed with one ml of 75 % methanol and then with 3 ml of 80 % methanol. The 80 % methanol eluate was run into the counting vial and 10 ml of scintillation liquid was added. Radioactivity was counted using a double-label program on a liquid scintillation counter (LKB RackBeta). Typical activities for placental and bovine adrenal enzyme preparation were 0,5-3 and 50-100 pmol pregnenolone formed/mg protein/min, respectively.

In inhibition experiments, the substance (final concentration range from 1 to 1000 µM) was added into incubation mixture in a volume of 10-20 µl, usually as methanol or ethanol solution. The same volume of the solute was added into control incubation vial. The IC-50 values (concentration causing a 50 % inhibition) were determined graphically and are presented in Table 2.

Table 1

| Compounds tested | |
|---|---|
| No. | Name |
| 1. | 4-(2,4-diphenylbutyl)-1H-imidazole |
| 2. | 4-[2-(4-fluorophenyl)-4-phenylbutyl]-1H-imidazole |
| 3. | 4-[2,4-bis(4-fluorophenyl)butyl]-1H-imidazole |
| 4. | 4-[2-(4-cyanophenyl)-4-(4-fluorophenyl)butyl]-1H-imidazole |

Table 2: Inhibition of human aromatase and desmolase by test compounds. IC-50 represents the concentration which inhibits the enzyme 50 %.

| Compound No. | AROMATASE IC-50 µmol/l | DESMOLASE IC-50 µmol/l |
|---|---|---|
| 1. | 5,5 | 12 |
| 2. | 2,2 | 28 |
| 3. | 1,3 | 20 |
| 4. | 0,5 | 6,6 |

The daily dose for a patient varies from about 20 to about 200 mg, administered orally.

Acute toxicity, $LD_{50}$, was determined by using young adult female mice of NMRI-strain. The administration was oral. The $LD_{50}$-value for 4-[2,4-bis(4-fluorophenyl)butyl]-1H-imidazole was 400 mg/kg.

The following examples illustrate the invention.

$^1H$ NMR spectra were determined with a Bruker AC-P300 apparatus. The reference substance was tetramethylsilane. MS spectra were determined with Kratos MS80RF Autoconsole apparatus.

Example 1

a) 1-benzyl-5-[2-(4-fluorophenyl)-4-phenyl-1-butenyl]-1H-imidazole

A flask is charged with Zn (41,7 g, 0,642 mol) and 200 ml of tetrahydrofuran. $TiCl_4$ (60,3 g, 0,321 mol) is added dropwise to the mixture at 0 °C-10 °C and then the mixture is refluxed for an hour. 12,2 g (0,054 mol) 4'-fluoropropiophenone and 14,9 g (0,080 mol) 1-benzyl-5-imidazolylaldehyde in 250 ml of THF is added to the mixture at room temperature. The mixture is heated to boiling and the refluxing is continued for three hours. After cooling to room temperature the mixture is poured to 10 % $K_2CO_3$-solution. Toluene is added and the mixture is filtered through siliceous earth. The toluene phase is separated and the water layer is extracted again with toluene. The toluene extracts are combined, washed with water, dried with $MgSO_4$ and evaporated to dryness. The crude product is purified by flash chromatography with methylene chloride and methanol (9,75:0,25) as eluent.

MS: 382(14, $M^{+\bullet}$), 291 (34), 200 (4), 91 (100)

Using the same method for example the following compounds included in the invention were prepared:
1-benzyl-5-(2,4-diphenyl-1-butenyl)-1H-imidazole
MS: 364 (30, $M^{+\bullet}$), 273 (72), 182 (9), 91 (100), 65 (11)
1-benzyl-5-[2,4-bis(4-fluorophenyl)-1-butenyl]-1H-imidazole
MS: 400 (31, $M^{+\bullet}$), 291 (73), 200 (9), 109 (35), 91 (100)
$^1$H NMR (as HCl-salt, $CDCl_3$):
2.62 (broken t, 2H), 2.82 (broken t, 2H), 5.34 (s, 2H), 6.20 (s, 1H), 6.43 (s, 1H), 6.86-7.5 (m, 13H), 8.93 (s, 1H)

b) 4-[2-(4-fluorophenyl)-4-phenylbutyl]-1H-imidazole

1-benzyl-5-[2-(4-fluorophenyl)-4-phenyl-1-butenyl]-1H-imidazole (4,58 g, 0,0012 mol) is dissolved in ethanol-water-solution (25:15). 0,46 g of 10 % Pd/C and 3,8 g (0,06 mol) of ammoniumformate in 15 ml of water is added to the mixture. After refluxing for 2 hours the mixture is filtered and the filtrate is evaporated to dryness. The residue is dissolved into methylene chloride and washed a few times with water. Methylene chloride is evaporated and the residue is dissolved into 2 M hydrogen chloride solution. The solution is extracted twice with diethyl ether. The water layer is made alkaline and extracted with methylene chloride. The methylene chloride phase is dried and evaporated to dryness. The product is then made to hydrochloride salt with dry hydrogen chloride gas in diethyl ether.
MS: 294 (12, $M^{+\bullet}$), 203 (36), 190 (28), 109 (28), 91 (100), 82 (42)
Using the same method for example the following compounds included in the invention were prepared:
4-(2,4-diphenylbutyl)-1H-imidazole
MS: 276 (14, $M^{+\bullet}$), 185 (31), 172 (24), 91 (100), 82 (43)
$^1$H NMR (as base, $CDCl_3$):
1.9-2.1 (m, 2H), 2.4-2.5 (m, 2H), 2.8-3.0 (m, 3H), 6.53 (s, 1H), 7.0-7.4 (m, 11H)
4-[2,4-bis(4-fluorophenyl)butyl]-1H-imidazole
MS: 312 (4, $M^{+\bullet}$), 203 (16), 190 (22), 109 (100), 91 (9), 81 (52)
$^1$H NMR (as HCl-salt, MeOH-$d_4$):
1.95-2.1 (m, 2H), 2.4-2.5 (m, 2H), 2.9-3.1 (m, 3H), 6.9-7.25 (m, 9H), 8.68 (s, 1H)

## Example 2

4-[2-(4-cyanophenyl)-4-(4-fluorophenyl)butyl]-1H-imidazole

a) 1-benzyl-5-[2-(4-cyanophenyl)-4-(4-fluorophenyl)-1-butenyl]-1H-imidazole

4,46 g of titanium tetrachloride is added dropwise to a stirred suspension of zinc powder (3,08 g) in tetrahydrofuran (50 ml) at -10 °C under dry nitrogen. The mixture is heated to reflux and refluxing is continued for one hour. The solution is cooled to room temperature and 1,0 g of 1-(4-cyanophenyl)-3-(4-fluorophenyl)-propan-1-one in 25 ml of tetrahydrofuran and 0,73 g of 1-benzyl-5-imidazolecarbaldehyde in 25 ml of tetrahydrofuran are added into the mixture, respectively. The mixture is stirred at room temperature for 2 hours and refluxed for 6 hours. The dark mixture is poured into water (60 ml), rendered alkaline with 10 % potassium carbonate solution and extracted with toluene. The toluene solution is filtered through siliceous earth, the filtrate is evaporated and the residue is purified by flash chromatography.
MS: 407 ($M^{+\bullet}$, 8), 298 (35), 109 (13), 91 (100)

b) 4-[2-(4-cyanophenyl)-4-(4-fluorophenyl)butyl]-1H-imidizole

1-benzyl-5-[2-(4-cyanophenyl)-4-(4-fluorophenyl)-1-butenyl]-1H-imidizole hydrochloride is dissolved in ethanol and a catalytic amount of 10 % Pd/C is added. The reaction mixture is agitated vigorously at room temperature in a hydrogen atmosphere until the reduction and the debenzylation are complete. The reaction mixture is filtered and evaporated to dryness. The product is purified by flash chromatography.
$^1$H NMR (as HCl-salt, MeOH-$d_4$):
2.0-2.15 (m, 2H), 2.46 (t, 2H), 3.0-3.16 (m, 3H), 6.96 (t, 2H), 7.0-7.11 (m, 3H), 7.38 (d, 2H), 7.68 (d, 2H), 8.70 (s, 1H)

## Claims

**Claims for the following Contracting States : AT, BE, CH, DE, DK, FR, GB, IT, LI, LU, NL, SE**

1. A compound which is a substituted imidazole of the formula:

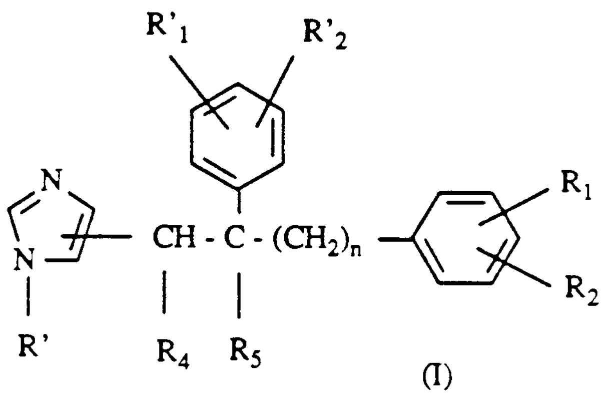

(I)

or a non-toxic pharmaceutically acceptable acid addition salt thereof wherein $R_1$, $R_2$, $R'_1$ and $R'_2$, which can be the same or different, are H, $CH_3$, $C_2H_5$, $C_3H_7$, $OCH_3$, $NO_2$, $NH_2$, CN, $CF_3$, $CHF_2$, $CH_2F$ or halogen; R' is H or

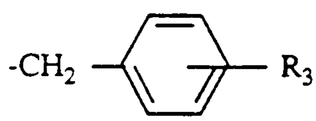

where $R_3$ is H, $CH_3$ or halogen; $R_4$ is H or OH and $R_5$ is H or OH or $R_4$ and $R_5$ together form a bond and n is 1 to 4.

2. A compound according to claim 1 wherein $R_4$ and $R_5$ both are H.

3. A compound according to claim 1 or 2 wherein $R_1$, $R_2$, $R'_1$ and $R'_2$ each are H.

4. A compound according to claim 1 or 2 wherein $R_1$ and $R'_1$ both are H and $R_2$ and $R'_2$, which are as defined in claim 1, both are in the para position of the phenyl group.

5. A compound according to any one of claims 1 to 4 wherein $R_2$ and $R'_2$, which can be the same or different, are CN or halogen.

6. A compound according to claim 5 wherein halogen is F.

7. A compound according to any one of claims 1 to 6 wherein R' is H.

8. A compound according to claim 1, which is 4-(2,4-diphenylbutyl)-1H-imidazole or a non-toxic pharmaceutically acceptable acid addition salt thereof.

9. A compound according to claim 1, which is 4-[2-(4-fluorophenyl)-4-phenylbutyl]-1H-imidazole or a non-toxic pharmaceutically acceptable acid addition salt thereof.

10. A compound according to claim 1, which is 4-[2,4-bis(4-fluorophenyl)butyl]-1H-imidazole or a non-toxic pharmaceutically acceptable acid addition salt thereof.

11. A compound according to claim 1, which is 4-[2-(4-cyanophenyl)-4-(4-fluorophenyl)butyl]-1H-imidazole or a non-toxic pharmaceutically acceptable acid addition salt thereof.

**12.** A pharmaceutical composition comprising a compound as claimed in any one of claims 1 to 11 and a pharmaceutically acceptable carrier.

**13.** A compound as defined in any one of claims 1 to 11 for use in a method of medical treatment of the human or animal body.

**14.** A compound as defined in claim 13 for use as an aromatase inhibitor.

**Claims for the following Contracting States : ES, GR**

**1.** A process for the preparation of a therapeutically active substituted imidazole of the formula

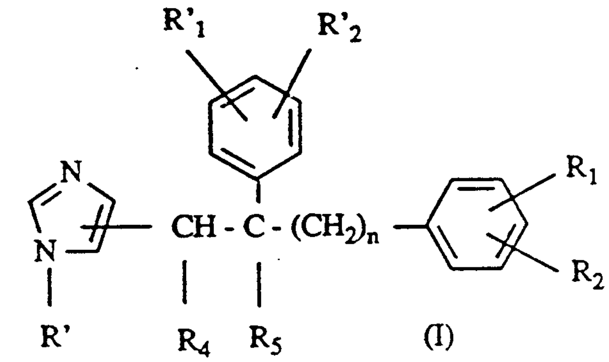

(I)

or a non-toxic pharmaceutically acceptable acid addition salt thereof wherein $R_1$, $R_2$, $R'_1$ and $R'_2$, which can be the same or different, are H, $CH_3$, $C_2H_5$, $C_3H_7$, $OCH_3$, $NO_2$, $NH_2$, CN, $CF_3$, $CHF_2$, $CH_2F$ or halogen; R' is H or

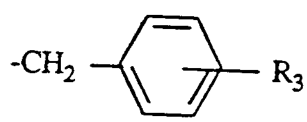

,

where $R_3$ is H, $CH_3$ or halogen; $R_4$ is H or OH and $R_5$ is H or OH or $R_4$ and $R_5$ together form a bond and n is 1 to 4 which comprises reacting a diphenylketone of the formula

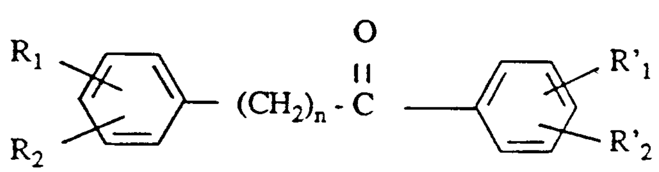

wherein $R_1$, $R_2$, $R'_1$ and $R'_2$, which can be the same or different, are H, $CH_3$, $C_2H_5$, $C_3H_7$, $OCH_3$, $NH_2$, CN, $CF_3$, $CHF_2$, $CH_2F$ or halogen and n is 1-4, with an 4(5)-imidazole aldehyde of the formula

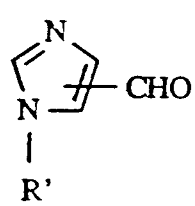

wherein R' is as defined before, in the presence of a low valent titanium reagent to give a compound of formula (I) which is

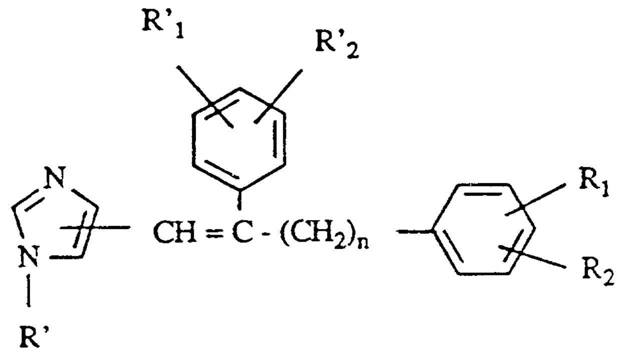

where R', $R_1$, $R_2$, $R'_1$, $R'_2$ and n are as defined above and if desired the resulting compound of formula (I) is transformed into a non-toxic pharmaceutically acceptable acid addition salt thereof.

**2.** A process for the preparation of a therapeutically active substituted imidazole of the formula (I) as defined in Claim 1 which process comprises reacting a ketone of the formula

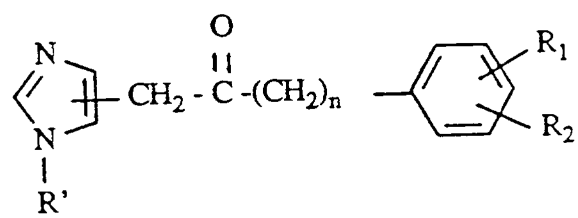

wherein R' and n are as defined in Claim 1 and $R_1$ and $R_2$, which can be the same or different, are H, $CH_3$, $C_2H_5$, $C_3H_7$, $OCH_3$, $NO_2$, $CF_3$, $CHF_2$, $CH_2F$ or halogen, with a halide derivative of the formula

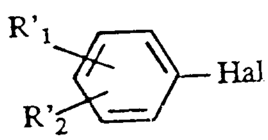

wherein $R'_1$ and $R'_2$, which can be the same or different, are H, $CH_3$, $C_2H_5$, $C_3H_7$, $OCH_3$, $NO_2$, $CF_3$, $CHF_2$, $CH_2F$ or halogen, in the presence of an alkyl lithium or magnesium to give a compound of formula

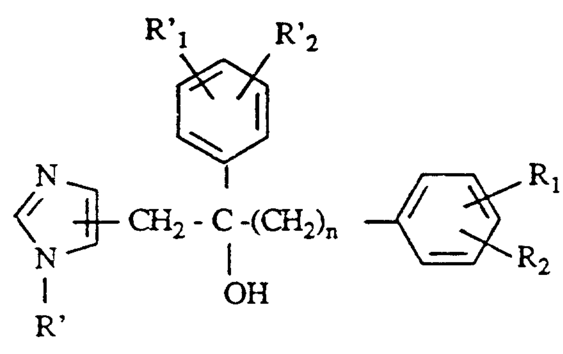

wherein R', $R_1$, $R_2$, $R'_1$, $R'_2$ and n are as defined in Claim 1 and if desired the resulting compound of formula (I) is transformed into a non-toxic pharmaceutically acceptable acid addition salt thereof.

**3.** A process for the preparation of a therapeutically active substituted imidazole of the formula (I) as defined in Claim 1 which process comprises dehydrating a compound of formula

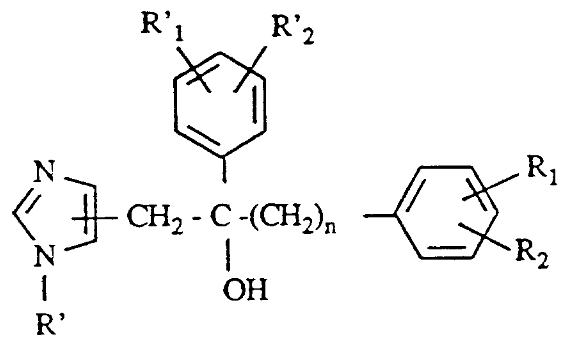

wherein R', $R_1$, $R_2$, $R'_1$, $R'_2$ and n are as defined in claim 2 to give a compound of formula (I) which is

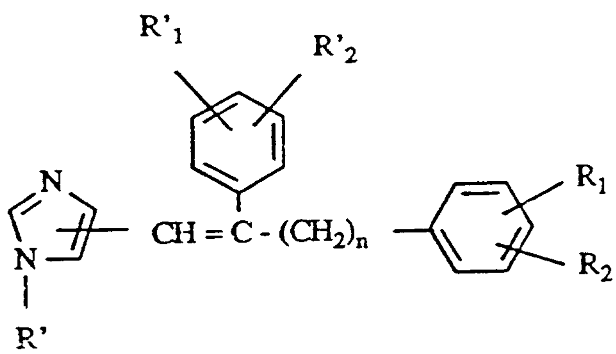

wherein R', $R_1$, $R_2$, $R'_1$, $R'_2$ and n are as defined in Claim 2 and if desired the resulting compound of formula (I) is transformed into a non-toxic pharmaceutically acceptable acid addition salt thereof.

**4.** A process for the preparation of a therapeutically active substituted imidazole of the formula (I) as defined in Claim 1 which process comprises reacting a compound of formula

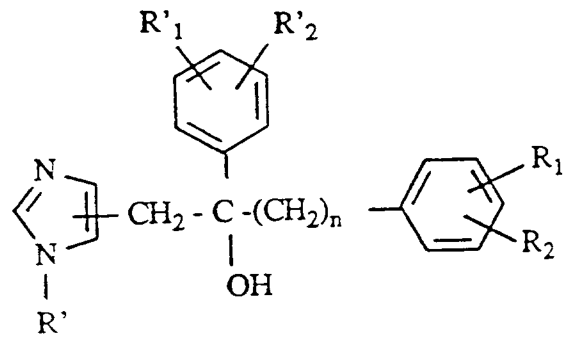

wherein R' is a protecting benzyl group and $R_1$, $R_2$, $R'_1$, $R'_2$ and n are as defined in Claim 2, by a hydrogen transfer reaction to give a compound of formula (I) which is

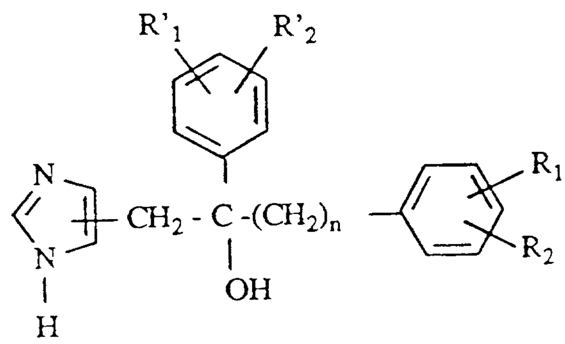

wherein $R_1$, $R_2$, $R'_1$, $R'_2$ and n are as defined in Claim 2 and if desired the resulting compound of formula (I) is transformed into a non-toxic pharmaceutically acceptable acid addition salt thereof.

**5.** A process for the preparation of a therapeutically active substituted imidazole of the formula (I) as defined in Claim 1 which process comprises reacting a diphenylhalogen of formula

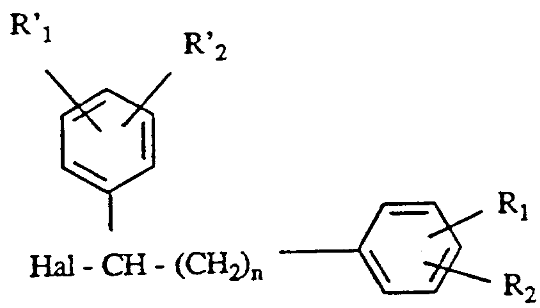

wherein $R_1$, $R_2$, $R'_1$, $R'_2$ and n are as defined in Claim 2 and Hal is halogen, with an 4(5)-imidazole aldehyde of formula

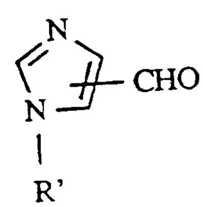

wherein R' is as defined in Claim 2, in the presence of n-butyllithium to give a compound of formula (I) which is

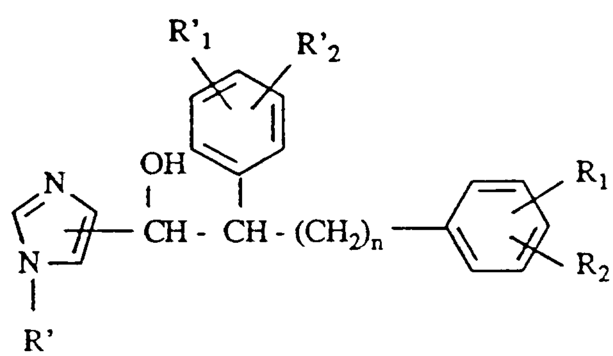

wherein R', $R_1$, $R_2$, $R'_1$, $R'_2$ and n are as defined in Claim 2 and if desired the resulting compound of formula (I) is transformed into a non-toxic pharmaceutically acceptable acid addition salt thereof.

6. A process for the preparation of a therapeutically active substituted imidazole of the formula (I) as defined in Claim 1 which process comprises dehydrating a compound of formula

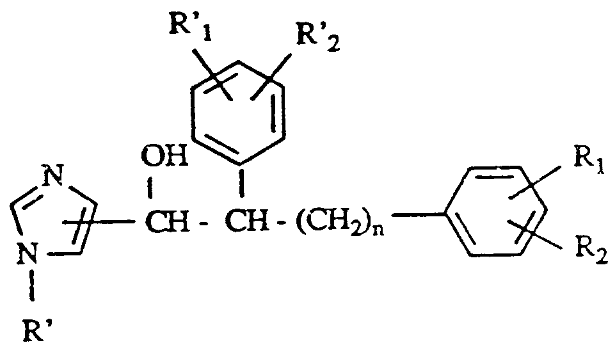

wherein R', $R_1$, $R_2$, $R'_1$, $R'_2$ and n are as defined in Claim 2 to give a compound of formula (I) which is

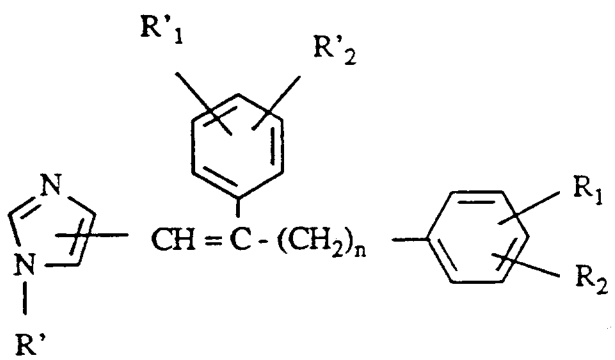

wherein R', $R_1$, $R_2$, $R'_1$, $R'_2$ and n are as defined in Claim 2 and if desired the resulting compound of formula (I) is transformed into a non-toxic pharmaceutically acceptable acid addition salt thereof.

7. A process for the preparation of a therapeutically active substituted imidazole of the formula (I) as defined in Claim 1 which process comprises reacting an aldehyde of formula

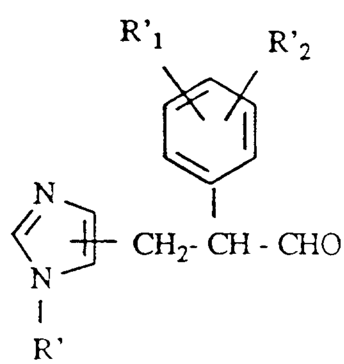

or an amide of formula

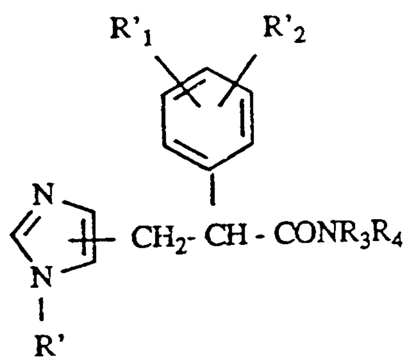

wherein $R'_1$ and $R'_2$, which can be the same or different, are H, $CH_3$, $C_2H_5$, $C_3H_7$, $OCH_3$, $NO_2$, $CF_3$, $CHF_2$, $CH_2F$ or halogen, R' is as defined in Claim 2 and $R_3$ and $R_4$ are lower alkyl or N, $R_3$ and $R_4$ together form a ring, with an appropriate aryl- or arylalkyl halide

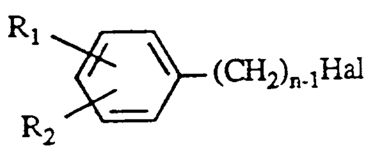

wherein $R_1$ and $R_2$, which can be the same or different, are H, $CH_3$, $C_2H_5$, $C_3H_7$, $OCH_3$, $NO_2$, $NH_2$, CN, $CF_3$, $CHF_2$, $CH_2F$ or halogen and n is 1 to 4, in the presence of an alkyl lithium or magnesium to give a compound of formula

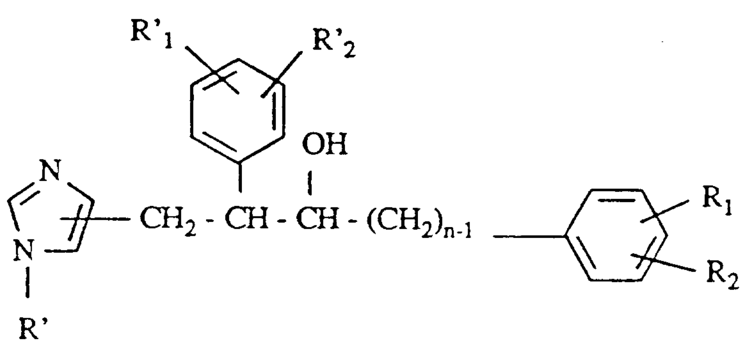

or

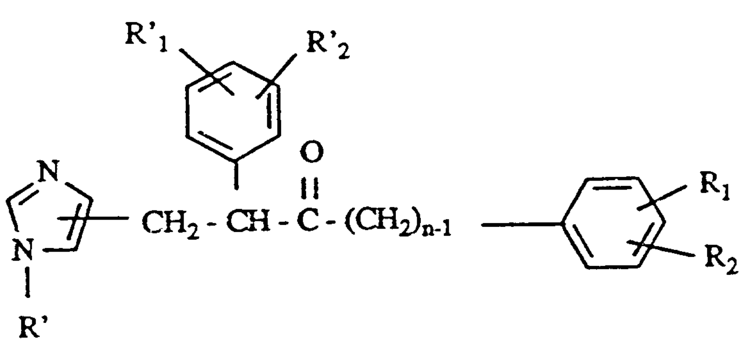

which is further reduced, dehydrated and hydrogenated to give a compound of formula (I) which is

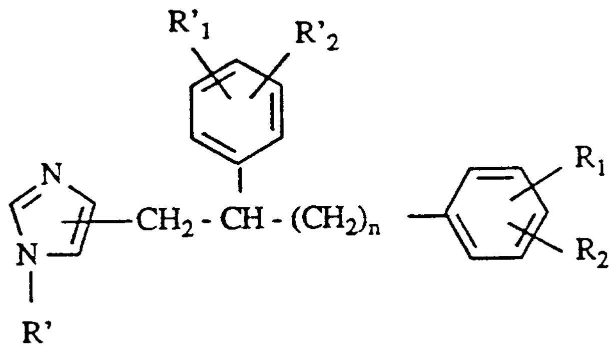

wherein R', and n are as defined above and $R_1$, $R_2$, $R'_1$ and $R'_2$, which can be the same or different, are H, $CH_3$, $C_2H_5$, $C_3H_7$, $OCH_3$, $NH_2$, CN, $CF_3$, $CHF_2$, $CH_2F$ or halogen and if desired the resulting compound of formula (I) is transformed into a non-toxic pharmaceutically acceptable acid addition salt thereof.

**8.** A process for the preparation of a therapeutically active substituted imidazole of the formula (I) as defined in Claim 1 which process comprises reacting a ketone of formula

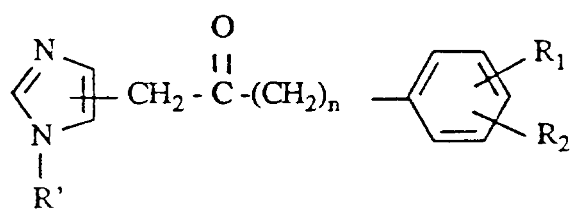

wherein R' is a protecting benzyl group, n is 1 to 4 with a halide derivative of the formula

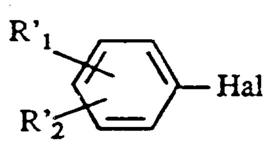

wherein Hal is halogen and one or more of the substituents $R_1$, $R_2$, $R'_1$ and $R'_2$ are a tert-butylaminocarbonyl group or an oxazoline group and the substituents which are not a tert-butylaminocarbonyl or an oxazoline group are H, $CH_3$, $C_2H_5$, $C_3H_7$, $OCH_3$, $CF_3$, $CHF_2$, $CH_2F$ or halogen, in the presence of an alkyl lithium or magnesium to give a compound of formula

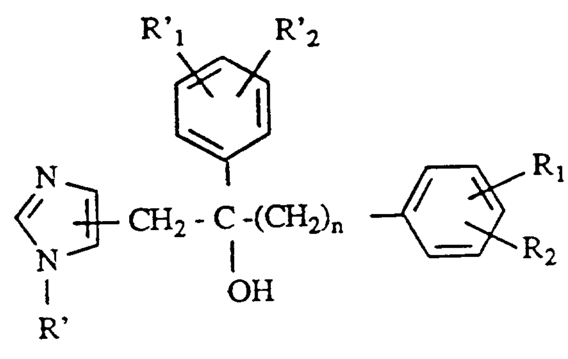

which is further dehydrated, hydrogenated and reacted with $SOCl_2$ to give a compound of formula (I) which is

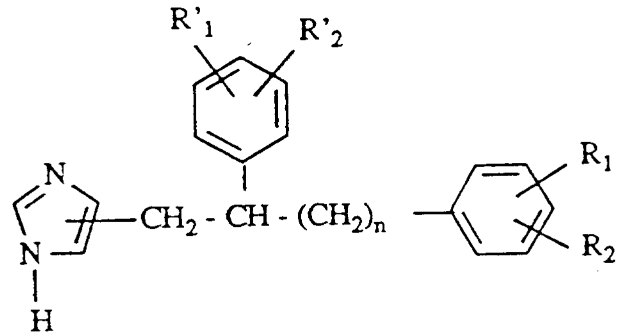

wherein one or more of the substituents $R_1$, $R_2$, $R'_1$ and $R'_2$ are CN and the substituents that are not CN are H, $CH_3$, $C_2H_5$, $C_3H_7$, $OCH_3$, $CF_3$, $CHF_2$, $CH_2F$ or halogen and if desired the resulting compound of formula (I) is transformed into a non-toxic pharmaceutically acceptable acid addition salt thereof.

**9.** A process for the preparation of a therapeutically active substituted imidazole of the formula (I) as defined in Claim 1 which process comprises reacting a compound of formula

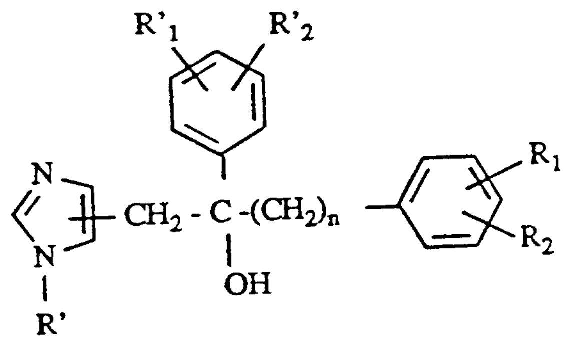

wherein R' is a protecting benzyl group and one or more of the substituents $R_1$, $R_2$, $R'_1$ and $R'_2$ are a tert-butylaminocarbonyl or an oxazole group and the substituents which are not a tert-butylaminocarbonyl or an oxazole group are as defined in Claim 8, with $SOCl_2$, $POCl_3$ or $PCl_5$ to give a compound of formula

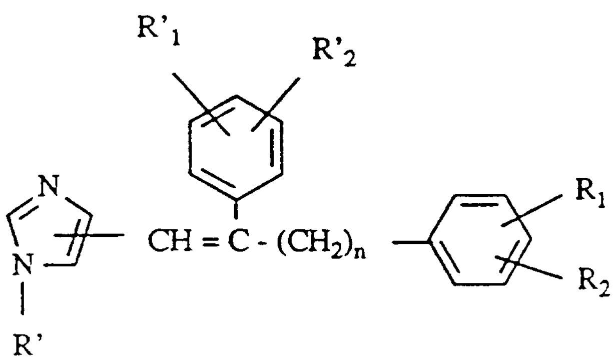

wherein R' is a protecting benzyl group, one or more of the substituents $R_1$, $R_2$, $R'_1$ and $R'_2$ are CN and the substituents that are not CN are as defined in Claim 8 and if desired the resulting compound of formula (I) is transformed into a non-toxic pharmaceutically acceptable acid addition salt thereof.

**10.** A process for the preparation of a therapeutically active substituted imidazole of the formula (I) as defined in Claim 1 which process comprises:

(a) diazotizing a compound of formula (I)

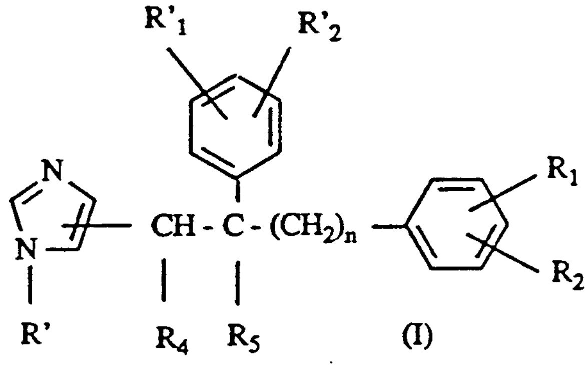

(I)

wherein R', $R_4$, $R_5$ and n are as defined in Claim 1 and one or more of the substituents $R_1$, $R_2$, $R'_1$ and $R'_2$ are $NH_2$ and the substituents which are not $NH_2$ are as defined in Claim 1 to give a compound of formula (I) wherein one or more of the substituents $R_1$, $R_2$, $R'_1$ and $R'_2$ are CN and the substituents which are not CN are as defined in Claim 1; or

(b) hydrogenating a compound of formula (I)

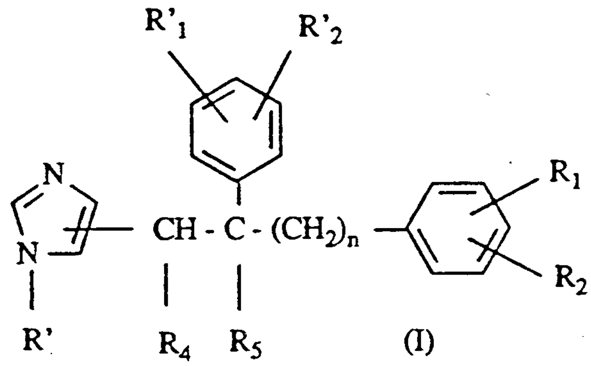

(I)

wherein R', $R_4$, $R_5$ and n are as defined in Claim 1 and one or more of the substituents $R_1$, $R_2$, $R'_1$ and $R'_2$ are $NO_2$ and the substituents which are not $NO_2$ are as defined in claim 1, to give a compound of formula (I) wherein R' is H, $R_4$, $R_5$ and n as defined above and one or more of the substituents $R_1$, $R_2$, $R'_1$ and $R'_2$ are $NH_2$ and the substituents which are not $NH_2$ are as defined in Claim 1; or

(c) nitration of a compound of formula (I)

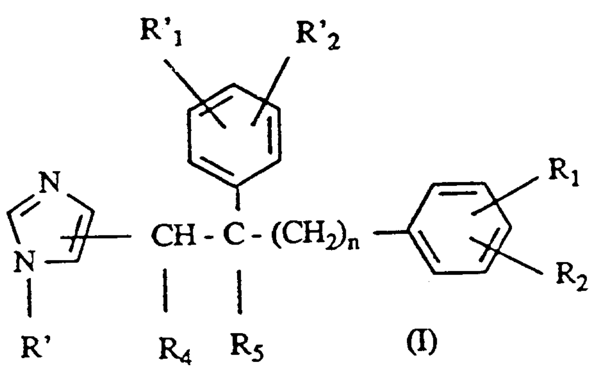

(I)

wherein R', $R_4$, $R_5$ and n are as defined before in Claim 1 and one or more of the substituents $R_1$, $R_2$, $R'_1$ and $R'_2$ are H and the substituents which are not H are as defined in claim 1, to give a

compound of formula (I) wherein one or more of the substituents $R_1$, $R_2$, $R'_1$ and $R'_2$ are $NO_2$ and the substituents which are not $NO_2$ are as defined in Claim 1;

and if desired the resulting compound of formula (I) is transformed into a non-toxic pharmaceutically acceptable acid addition salt thereof.

**11.** A process for the preparation of a therapeutically active substituted imidazole of the formula (I) as defined in Claim 1 which process comprises:

(a) hydrogenating a compound of formula

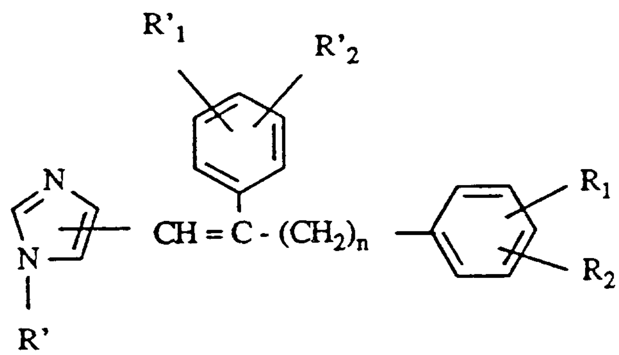

wherein R' and n are as defined in Claim 1 and $R_1$, $R_2$, $R'_1$ and $R'_2$, which can be the same or different, are H, $CH_3$, $C_2H_5$, $C_3H_7$, $OCH_3$, $NO_2$, $NH_2$, CN, $CF_3$, $CHF_2$, $CH_2F$ or halogen, to give a compound of formula (I) which is

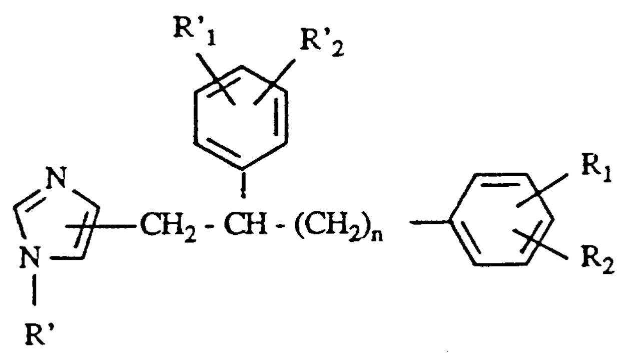

wherein R', $R_1$, $R_2$, $R'_1$ and $R'_2$ and n are as defined above; or

(b) hydrogenating a compound of formula

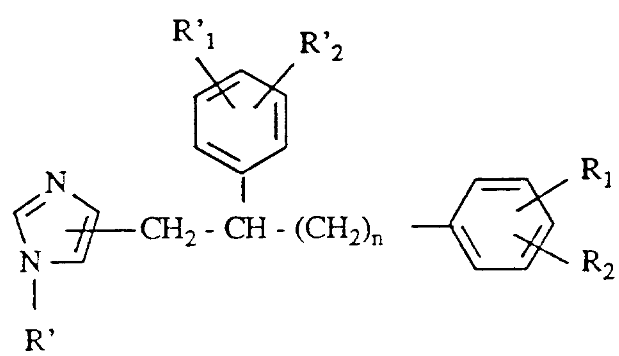

wherein $R_1$, $R_2$, $R'_1$, $R'_2$ and n are as defined above and R' is a substituted or unsubstituted benzyl group, to give a compound of formula (I) which is

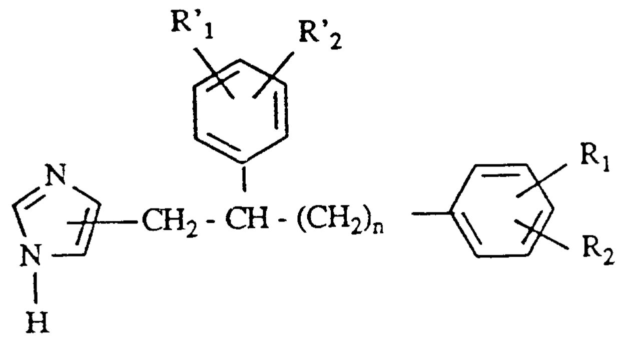

wherein $R_1$, $R_2$, $R'_1$, $R'_2$ and n are as defined above;
and if desired the resulting compound of formula (I) is transformed into a non-toxic pharmaceutically acceptable acid addition salt thereof.

**12.** A process for the preparation of a therapeutically active substituted imidazole of the formula (I) as defined in Claim 1 which process comprises hydrogenating a compound of formula

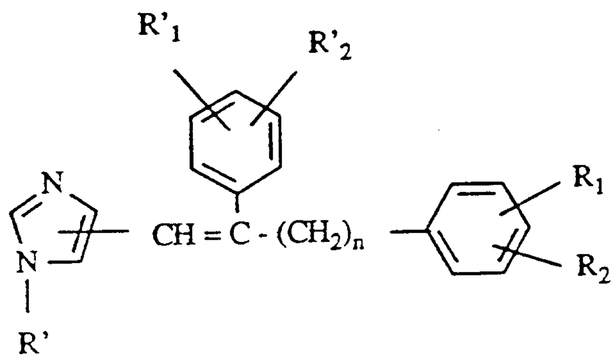

wherein R', $R_1$, $R_2$, $R'_1$, $R'_2$ and n are as defined in Claim 11 to give a compound of formula (I) which is

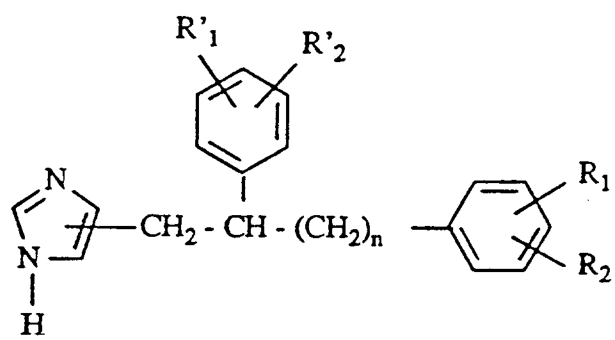

wherein $R_1$, $R_2$, $R'_1$, $R'_2$ and n are as defined in Claim 11 and if desired the resulting compound of formula (I) is transformed into a non-toxic pharmaceutically acceptable acid addition salt thereof.

**13.** A process for the preparation of a therapeutically active substituted imidazole of the formula (I) as defined in Claim 1 which process comprises reacting a compound of formula

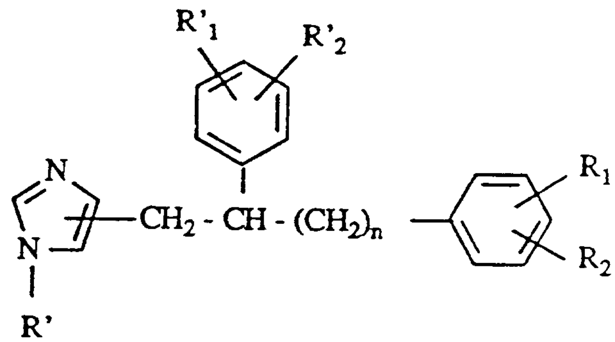

wherein R' is a substituted or unsubstituted benzyl group, n is 1 to 4 and $R_1$, $R_2$, $R'_1$ and $R'_2$, which can be the same or different, are H, $CH_3$, $C_2H_5$, $C_3H_7$, $OCH_3$, $NO_2$, $NH_2$, $CF_3$, $CHF_2$, $CH_2F$ or halogen, by a hydrogen transfer reaction to give a compound of formula (I) which is

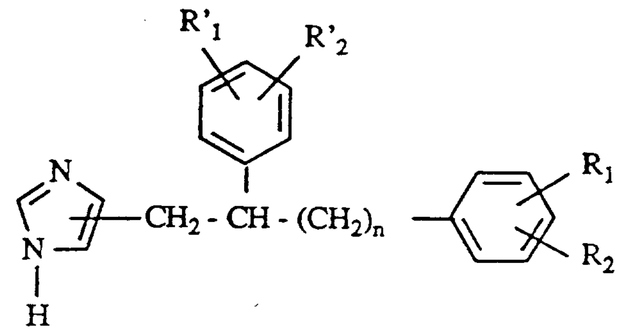

wherein $R_1$, $R_2$, $R'_1$ and $R'_2$ and n are as defined above and if desired the resulting compound of formula (I) is transformed into a non-toxic pharmaceutically acceptable acid addition salt thereof.

**14.** A process for the preparation of a therapeutically active substituted imidazole of the formula (I) as defined in Claim 1 which process comprises reacting a compound of formula

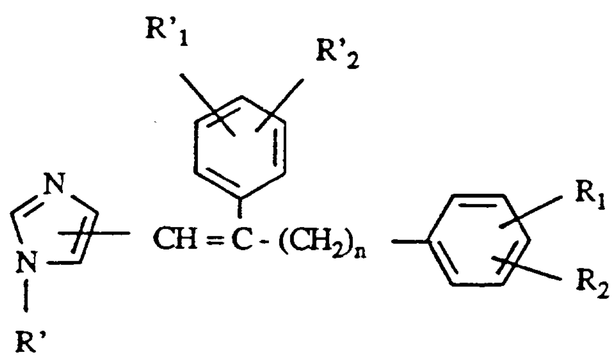

wherein R' and n are as defined in Claim 1 and $R_1$, $R_2$, $R'_1$ and $R'_2$ are as defined in Claim 13, by a hydrogen transfer reaction to give a compound of formula (I) which is

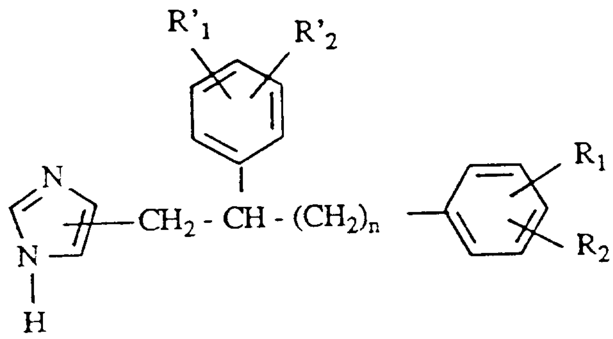

wherein $R_1$, $R_2$, $R'_1$ and $R'_2$ are as defined in claim 13 and if desired the resulting compound of formula (I) is transformed into a non-toxic pharmaceutically acceptable acid addition salt thereof.

**Patentansprüche**
**Patentansprüche für folgende Vertragsstaaten : AT, BE, CH, DE, DK, FR, GB, IT, LI, LU, NL, SE**

1. Verbindung, die ein substituiertes Imidazol der Formel

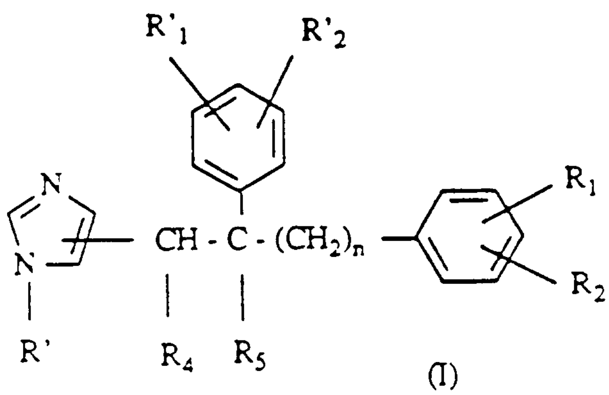

(I)

ist, oder deren nicht-toxisches, pharmazeutisch annehmbares Säureadditionssalz, worin $R_1$, $R_2$, $R'_1$ und $R'_2$, die gleich oder verschieden sein können, H, $CH_3$, $C_2H_5$, $C_3H_7$, $OCH_3$, $NO_2$, $NH_2$, CN, $CF_3$, $CHF_2$, $CH_2F$ oder Halogen sind, R' H oder

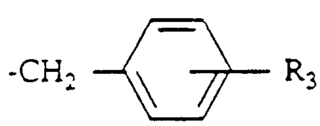

ist, worin $R_3$ H, $CH_3$ oder Halogen bedeutet, $R_4$ H oder OH ist und $R_5$ H oder OH ist oder $R_4$ und $R_5$ zusammen eine Bindung bilden und n 1 bis 4 ist.

2. Verbindung gemäß Anspruch 1, worin $R_4$ und $R_5$ beide H sind.

3. Verbindung gemäß Anspruch 1 oder 2, worin $R_1$, $R_2$, $R'_1$ und $R'_2$ alle H sind.

4. Verbindung gemäß Anspruch 1 oder 2, worin $R_1$ und $R'_1$ beide H sind und $R_2$ und $R'_2$, die wie in Anspruch 1 definiert sind, sich beide in para-Stellung der Phenylgruppe befinden.

5. Verbindung gemäß irgendeinem der Ansprüche 1 bis 4, worin $R_2$ und $R'_2$, die gleich oder verschieden sein können, CN oder Halogen sind.

6. Verbindung gemäß Anspruch 5, worin Halogen F ist.

7. Verbindung gemäß irgendeinem der Ansprüche 1 bis 6, worin R' H ist.

8. Verbindung gemäß Anspruch 1, welche 4-(2,4-Diphenylbutyl)-1H-imidazol oder ein nicht-toxisches, pharmazeutisch annehmbares Säureadditionssalz desselben ist.

9. Verbindung gemäß Anspruch 1, welche 4-[2-(4-Fluorphenyl)-4-phenylbutyl]-1H-imidazol oder ein nicht-toxisches, pharmazeutisch annehmbares Säureadditionssalz desselben ist.

10. Verbindung gemäß Anspruch 1, welche 4-[2,4-Bis(4-fluorphenyl)butyl]-1H-imidazol oder ein nicht-toxisches, pharmazeutisch annehmbares Säureadditionssalz desselben ist.

11. Verbindung gemäß Anspruch 1, welche 4-[2-(4-Cyanophenyl)-4-(4-fluorphenyl)butyl]-1H-imidazol oder ein nicht-toxisches, pharmazeutisch annehmbares Säureadditionssalz desselben ist.

12. Pharmazeutische Zusammensetzung, umfassend eine Verbindung wie in irgendeinem der Ansprüche 1 bis 11 beansprucht und einen pharmazeutisch annehmbaren Träger.

13. Verbindung wie in irgendeinem der Ansprüche 1 bis 11 definiert für die Verwendung in einem Verfahren der medizinischen Behandlung des menschlichen oder tierischen Körpers.

14. Verbindung wie in Anspruch 13 definiert für die Verwendung als Aromatase-Inhibitor.

**Patentansprüche für folgende Vertragsstaaten : ES, GR**

1. Verfahren zur Herstellung eines therapeutisch aktiven substituierten Imidazols der Formel

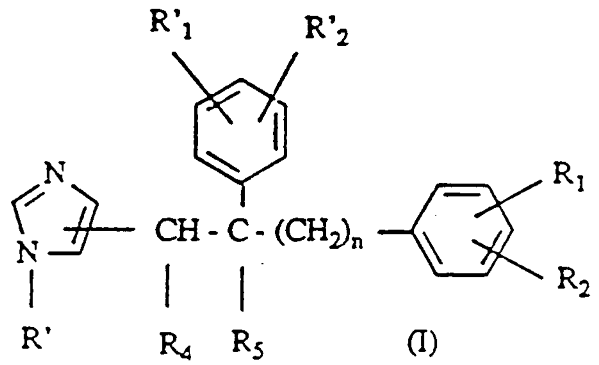

(I)

oder eines nicht-toxischen, pharmazeutisch annehmbaren Säureadditionssalzes desselben, worin $R_1$, $R_2$, $R'_1$ und $R'_2$, die gleich oder verschieden sein können, H, $CH_3$, $C_2H_5$, $C_3H_7$, $OCH_3$, $NO_2$, $NH_2$, CN, $CF_3$, $CHF_2$, $CH_2F$ oder Halogen sind, R' H oder

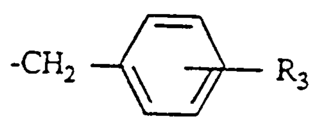

ist, worin $R_3$ H, $CH_3$ oder Halogen bedeutet, $R_4$ H oder OH ist und $R_5$ H oder OH ist oder $R_4$ und $R_5$ zusammen eine Bindung bilden und n 1 bis 4 ist,
welches umfaßt:
Umsetzen eines Diphenylketons der Formel

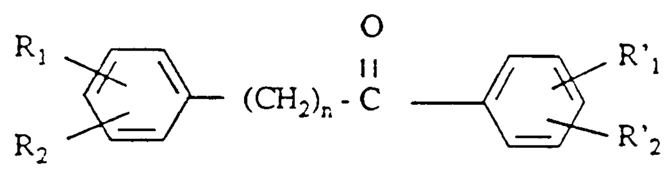

worin $R_1$, $R_2$, $R'_1$ und $R'_2$, die gleich oder verschieden sein können, H, $CH_3$, $C_2H_5$, $C_3H_7$, $OCH_3$, $NH_2$, CN, $CF_3$, $CHF_2$, $CH_2F$ oder Halogen sind und n 1 bis 4 ist, mit einem 4(5)-Imidazolaldehyd der Formel

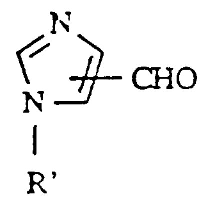

worin R' wie oben definiert ist, in Gegenwart eines niedervalenten Titanreagenz, um eine Verbindung der Formel (I) zu ergeben, die

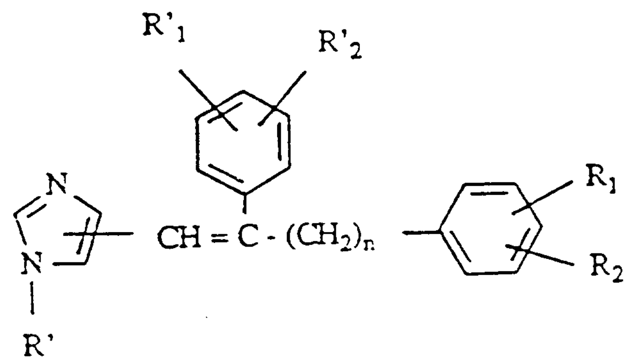

ist, worin R', $R_1$, $R_2$, $R'_1$, $R'_2$ und n wie oben definiert sind, und, falls gewünscht, die resultierende Verbindung der Formel (I) in ein nicht-toxisches, pharmazeutisch annehmbares Säureadditionssalz derselben überführt wird.

**2.** Verfahren zur Herstellung eines therapeutisch aktiven substituierten Imidazols der Formel (I) wie in Anspruch 1 definiert, worin das Verfahren umfaßt:
Umsetzen eines Ketons der Formel

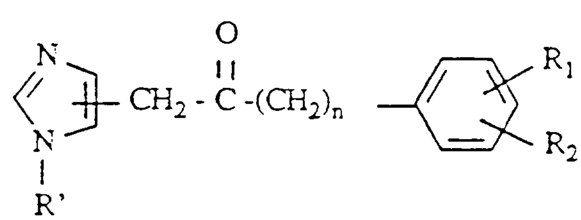

worin R' und n wie in Anspruch 1 definiert sind und $R_1$ und $R_2$, die gleich oder verschieden sein können, H, $CH_3$, $C_2H_5$, $C_3H_7$, $OCH_3$, $NO_2$, $CF_3$, $CHF_2$, $CH_2F$ oder Halogen sind, mit einem Halogenderivat der Formel

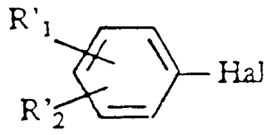

worin R'$_1$ und R'$_2$, die gleich oder verschieden sein können, H, $CH_3$, $C_2H_5$, $C_3H_7$, $OCH_3$, $NO_2$, $CF_3$, $CHF_2$, $CH_2F$ oder Halogen sind, in Gegenwart eines Alkyllithiums oder -magnesiums, um eine Verbindung der Formel

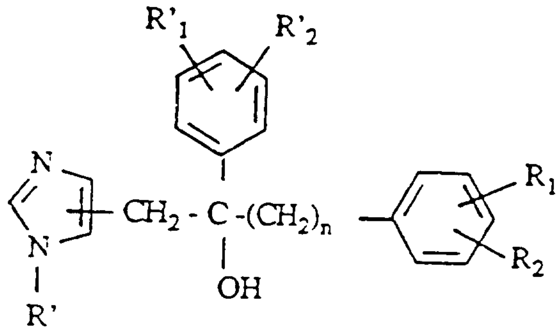

worin R', $R_1$, $R_2$, R'$_1$, R'$_2$ und n wie in Anspruch 1 definiert sind, und, falls gewünscht, Überführen der resultierenden Verbindung der Formel (I) in ein nicht-toxisches, pharmazeutisch annehmbares Säureadditionssalz derselben.

**3.** Verfahren zur Herstellung eines therapeutisch aktiven substituierten Imidazols der Formel (I) wie in Anspruch 1 definiert, welches umfaßt:
Dehydratisieren einer Verbindung der Formel

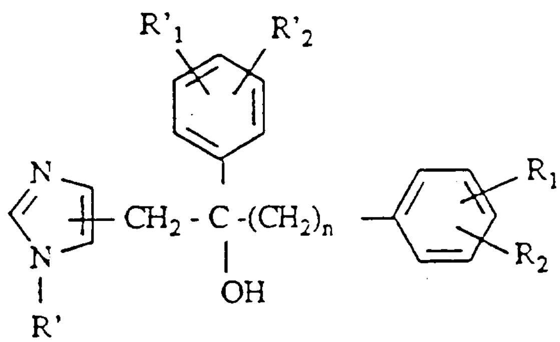

worin R', $R_1$, $R_2$, R'$_1$, R'$_2$ und n wie in Anspruch 2 definiert sind, um eine Verbindung der Formel (I) zu ergeben, die

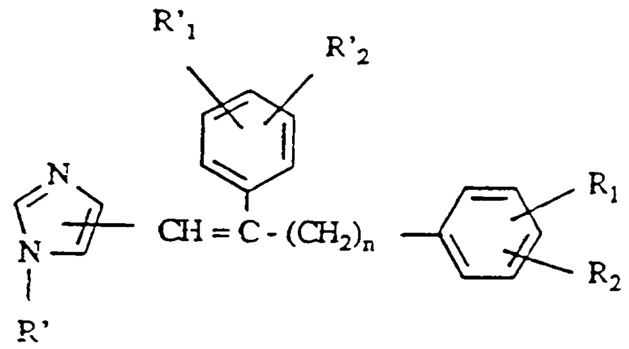

ist, worin R', $R_1$, $R_2$, $R'_1$, $R'_2$ und n wie in Anspruch 2 definiert sind, und, falls gewünscht, Überführen der resultierenden Verbindung der Formel (I) in ein nicht-toxisches, pharmazeutisch annehmbares Säureadditionssalz derselben.

**4.** Verfahren zur Herstellung eines therapeutisch aktiven substituierten Imidazols der Formel (I) wie in Anspruch 1 definiert, welches umfaßt:
Umsetzen einer Verbindung der Formel

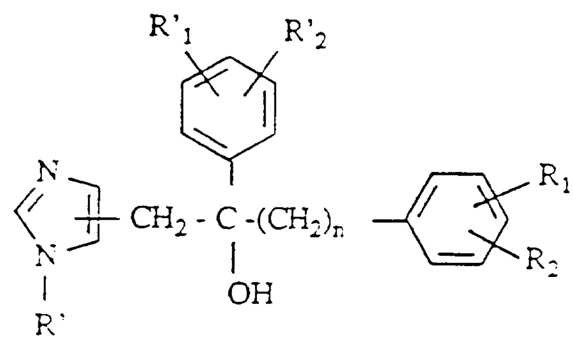

worin R' eine Benzyl-Schutzgruppe ist und $R_1$, $R_2$, $R'_1$, $R'_2$ und n wie in Anspruch 2 definiert sind, durch Wasserstoffübertragungsreaktion, um eine Verbindung der Formel (I) zu ergeben, die

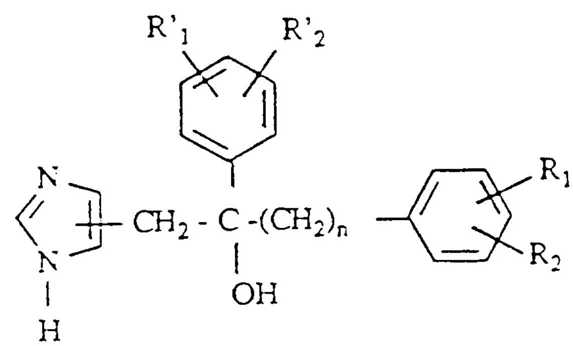

ist, worin $R_1$, $R_2$, $R'_1$, $R'_2$ und n wie in Anspruch 2 definiert sind, und, falls gewünscht, Überführen der resultierenden Verbindung der Formel (I) in ein nicht-toxisches, pharmazeutisch annehmbares Säureadditionssalz derselben.

**5.** Verfahren zur Herstellung eines therapeutisch aktiven substituierten Imidazols der Formel (I) wie in Anspruch 1 definiert, welches umfaßt:
Umsetzen eines Diphenylhalogens der Formel

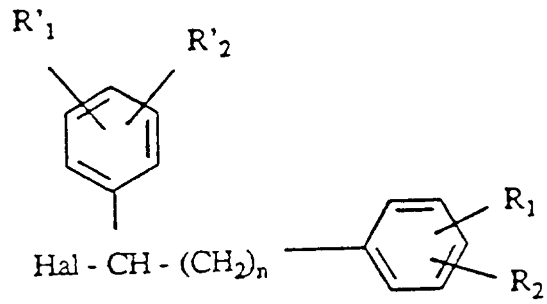

worin $R_1$, $R_2$, $R'_1$, $R'_2$ und n wie in Anspruch 2 definiert sind und Hal Halogen bedeutet, mit einem 4(5)-Imidazolaldehyd der Formel

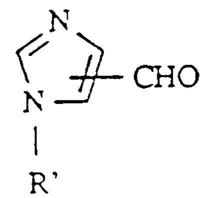

worin R' wie in Anspruch 2 definiert ist, in Gegenwart von n-Butyllithium, um eine Verbindung der Formel (I) zu ergeben, die

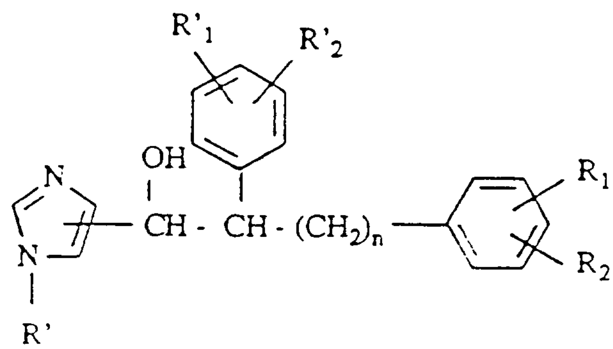

ist, worin R', $R_1$, $R_2$, $R'_1$, $R'_2$ und n wie in Anspruch 2 definiert sind, und, falls gewünscht, Überführen der resultierenden Verbindung der Formel (I) in ein nicht-toxisches, pharmazeutisch annehmbares Säureadditionssalz derselben.

**6.** Verfahren zur Herstellung eines therapeutisch aktiven substituierten Imidazols der Formel (I) wie in Anspruch 1 definiert, welches umfaßt:
Dehydratisieren einer Verbindung der Formel

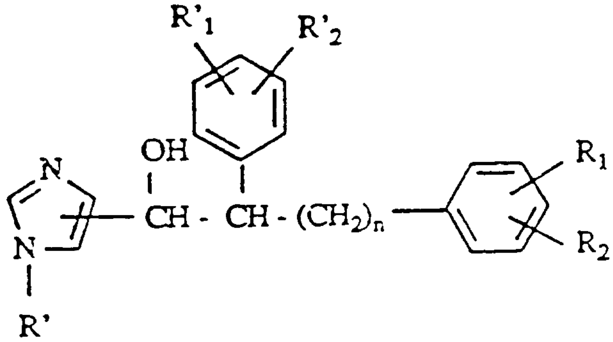

worin R', $R_1$, $R_2$, $R'_1$, $R'_2$ und n wie in Anspruch 2 definiert sind, um eine Verbindung der Formel (I) zu ergeben, die

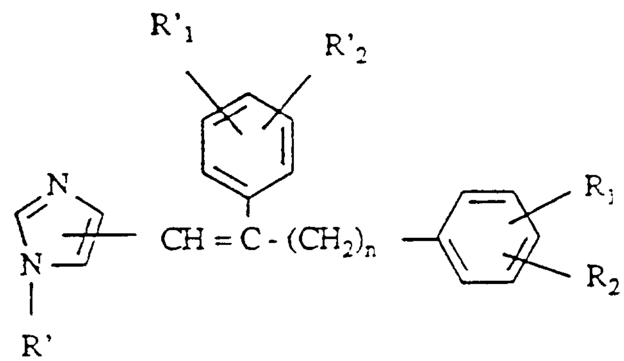

ist, worin R', $R_1$, $R_2$, $R'_1$, $R'_2$ und n wie in Anspruch 2 definiert sind, und, falls gewünscht, Überführen der resultierenden Verbindung der Formel (I) in ein nicht-toxisches, pharmazeutisch annehmbares Säureadditionssalz derselben.

7. Verfahren zur Herstellung eines therapeutisch aktiven substituierten Imidazols der Formel (I) wie in Anspruch 1 definiert, welches umfaßt:
Umsetzen eines Aldehyds der Formel

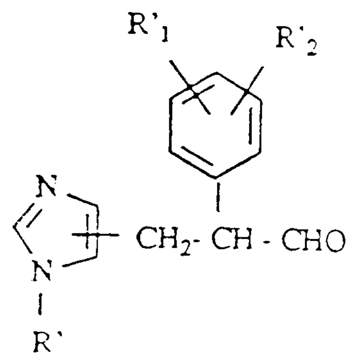

oder eines Amids der Formel

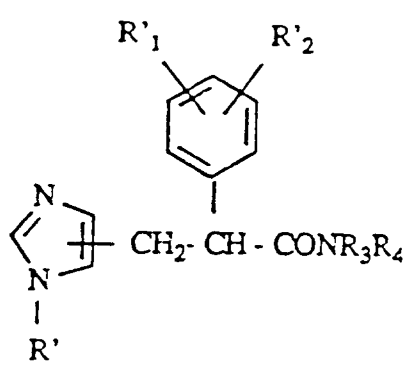

worin $R'_1$ und $R'_2$, die gleich oder verschieden sein können, H, $CH_3$, $C_2H_5$, $C_3H_7$, $OCH_3$, $NO_2$, $CF_3$, $CHF_2$, $CH_2F$ oder Halogen sind, R' wie in Anspruch 2 definiert ist und $R_3$ und $R_4$ Niederalkyl sind oder N, $R_3$ und $R_4$ zusammen einen Ring bilden, mit einem geeigneten Aryl- oder Arylalkylhalogenid

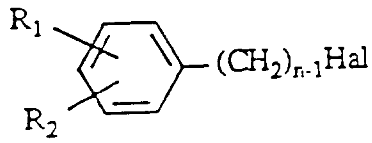

worin $R_1$ und $R_2$, die gleich oder verschieden sein können, H, $CH_3$, $C_2H_5$, $C_3H_7$, $OCH_3$, $NO_2$, $NH_2$, CN, $CF_3$, $CHF_2$, $CH_2F$ oder Halogen sind und n 1 bis 4 ist, in Gegenwart eines Alkyllithiums oder -magnesiums, um eine Verbindung der Formel

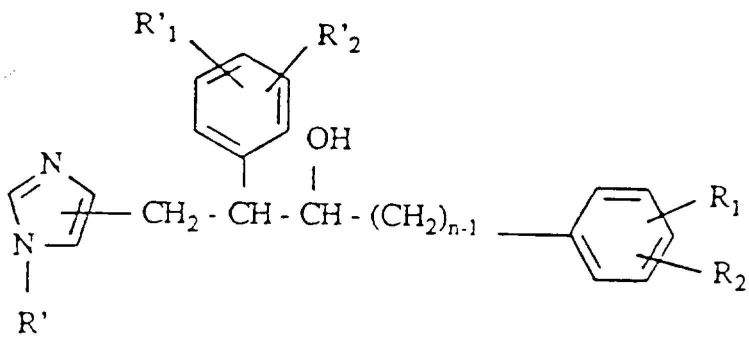

oder

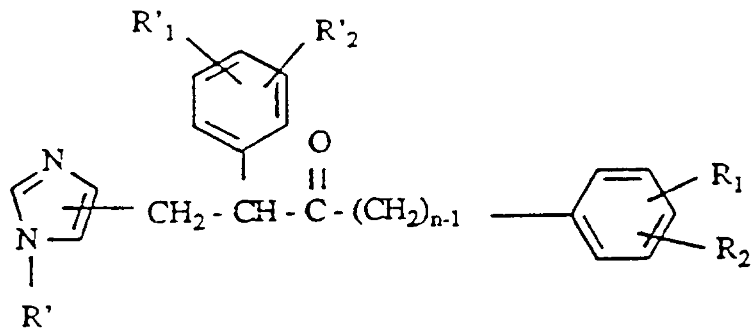

zu ergeben, die weiter reduziert, dehydratisiert und hydriert wird, um eine Verbindung der Formel (I) zu ergeben, die

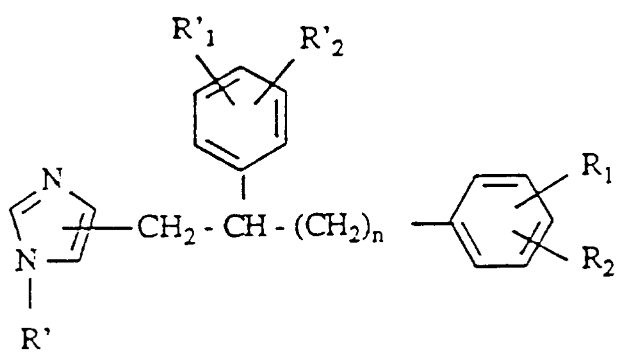

ist, worin R' und n wie oben definiert sind und $R_1$, $R_2$, $R'_1$ und $R'_2$, die gleich oder verschieden sein können, H, $CH_3$, $C_2H_5$, $C_3H_7$, $OCH_3$, $NH_2$, CN, $CF_3$, $CHF_2$, $CH_2F$ oder Halogen sind, und, falls gewünscht, Überführen der resultierenden Verbindung der Formel (I) in ein nicht-toxisches, pharmazeutisch annehmbares Säureadditionssalz derselben.

8. Verfahren zur Herstellung eines therapeutisch aktiven substituierten Imidazols der Formel (I) wie in Anspruch 1 definiert, welches umfaßt:
Umsetzen eines Ketons der Formel

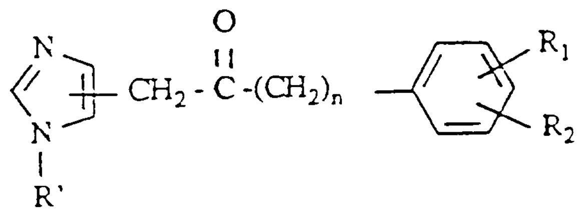

worin R' eine Benzyl-Schutzgruppe und n 1 bis 4 ist, mit einem Halogenderivat der Formel

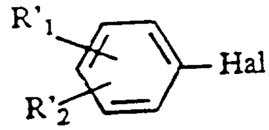

worin Hal Halogen ist und einer oder mehrere Substituenten $R_1$, $R_2$, $R'_1$ und $R'_2$ eine tert-Butylaminocarbonylgruppe oder eine Oxazolingruppe und die Substituenten, die keine tert-Butylaminocarbonylgruppe oder Oxazolingruppe sind, H, $CH_3$, $C_2H_5$, $C_3H_7$, $OCH_3$, $CF_3$, $CHF_2$, $CH_2F$ oder Halogen sind, in Gegenwart eines Alkyllithiums oder -magnesiums, um eine Verbindung der Formel

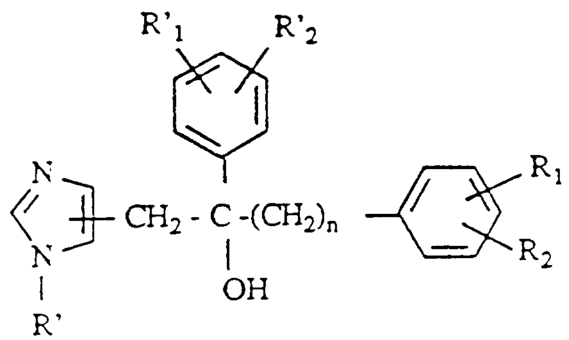

zu ergeben, die weiter dehydriert, hydriert und mit $SOCl_2$ umgesetzt wird, um eine Verbindung der Formel (I) zu ergeben, die

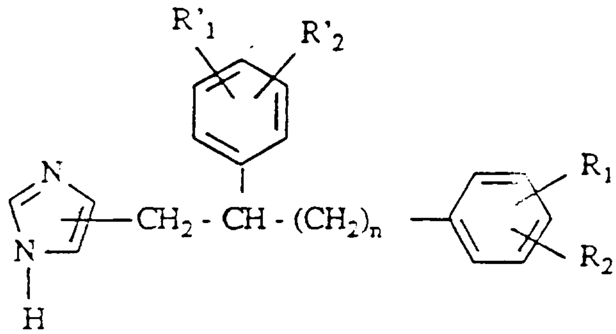

ist, worin einer oder mehrere der Substituenten $R_1$, $R_2$, $R'_1$ und $R'_2$ CN sind und die Substituenten, die nicht CN sind, H, $CH_3$, $C_2H_5$, $C_3H_7$, $OCH_3$, $CF_3$, $CHF_2$, $CH_2F$ oder Halogen sind, und, falls gewünscht, Überführen der resultierenden Verbindung der Formel (I) in ein nicht-toxisches, pharmazeutisch

annehmbares Säureadditionssalz derselben.

**9.** Verfahren zur Herstellung eines therapeutisch aktiven substituierten Imidazols der Formel (I) wie in Anspruch 1 definiert, welches umfaßt:
Umsetzen einer Verbindung der Formel

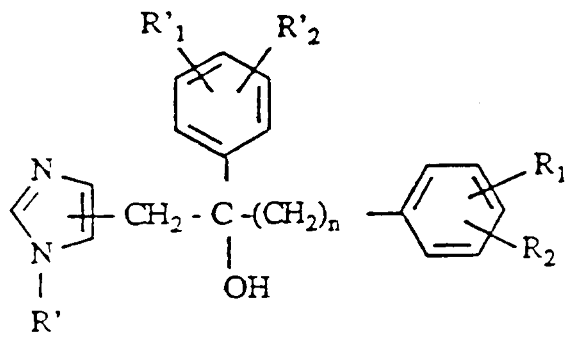

worin R' eine Benzyl-Schutzgruppe ist und einer oder mehrere der Substituenten $R_1$, $R_2$, $R'_1$ und $R'_2$ eine tert-Butylaminocarbonylgruppe oder eine Oxazolgruppe und die Substituenten, die keine tert-Butylaminocarbonylgruppe oder Oxazolgruppe sind, wie in Anspruch 8 definiert sind, mit $SOCl_2$, $POCl_3$ oder $PCl_5$, um eine Verbindung der Formel

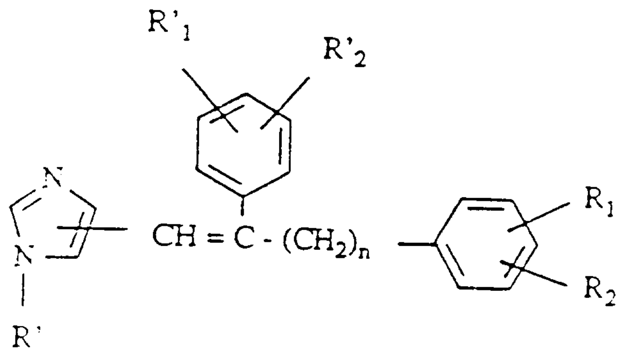

zu ergeben, worin R' eine Benzyl-Schutzgruppe ist, einer oder mehrere der Substituenten $R_1$, $R_2$, $R'_1$ und $R'_2$ CN sind und die Substituenten, die nicht CN sind, wie in Anspruch 8 definiert sind, und, falls gewünscht, Überführen der resultierenden Verbindung der Formel (I) in ein nicht-toxisches, pharmazeutisch annehmbares Säureadditionssalz derselben.

**10.** Verfahren zur Herstellung eines therapeutisch aktiven substituierten Imidazols der Formel (I) wie in Anspruch 1 definiert, welches umfaßt:

(a) Diazotieren einer Verbindung der Formel (I)

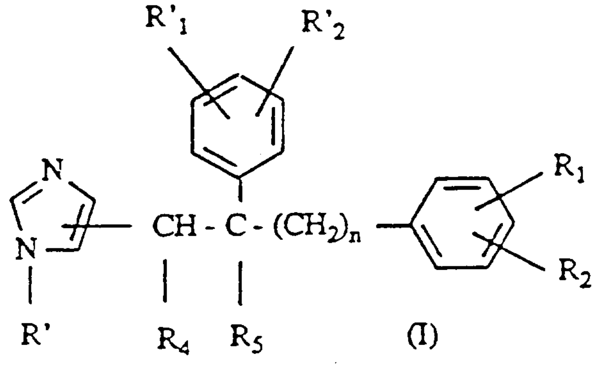

(I)

worin R', $R_4$, $R_5$ und n wie in Anspruch 1 definiert sind und einer oder mehrere der Substituenten $R_1$, $R_2$, $R'_1$ und $R'_2$ $NH_2$ sind und die Substituenten, die nicht $NH_2$ sind, wie in Anspruch 1 definiert sind, um eine Verbindung der Formel (I) zu ergeben, worin einer oder mehrere der Substituenten $R_1$, $R_2$, $R'_1$ und $R'_2$ CN sind und die Substituenten, die nicht CN sind, wie in Anspruch 1 definiert sind, oder
(b) Hydrieren einer Verbindung der Formel (I)

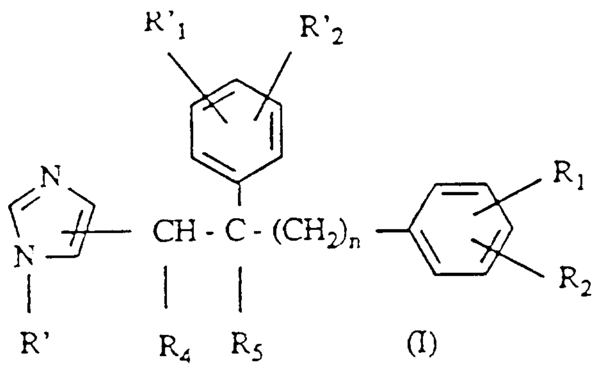

(I)

worin R', $R_4$, $R_5$ und n wie in Anspruch 1 definiert sind und einer oder mehrere der Substituenten $R_1$, $R_2$, $R'_1$ und $R'_2$ $NO_2$ sind und die Substituenten, die nicht $NO_2$ sind, wie in Anspruch 1 definiert sind, um eine Verbindung der Formel (I) zu ergeben, worin R' H ist, $R_4$, $R_5$ und n wie oben definiert sind und einer oder mehrere der Substituenten $R_1$, $R_2$, $R'_1$ und $R'_2$ $NH_2$ sind und die Substituenten, die nicht $NH_2$ sind, wie in Anspruch 1 definiert sind, oder
(c) Nitrieren einer Verbindung der Formel (I)

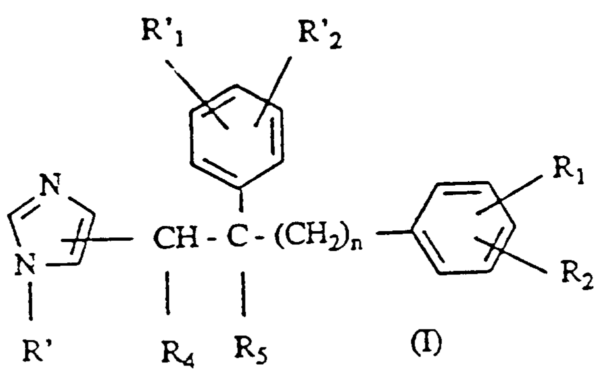

(I)

worin R', $R_4$, $R_5$ und n wie in Anspruch 1 definiert sind und einer oder mehrere der Substituenten $R_1$, $R_2$, $R'_1$ und $R'_2$ H sind und die Substituenten, die nicht H sind, wie in Anspruch 1 definiert sind, um eine Verbindung der Formel (I) zu ergeben, worin einer oder mehrere der Substituenten $R_1$, $R_2$, $R'_1$ und $R'_2$ $NO_2$ sind und die Substituenten, die nicht $NO_2$ sind, wie in Anspruch 1 definiert sind,
und, falls gewünscht, Überführen der resultierenden Verbindung der Formel (I) in ein nicht-toxisches, pharmazeutisch annehmbares Säureadditionssalz derselben.

**11.** Verfahren zur Herstellung eines therapeutisch aktiven substituierten Imidazols der Formel (I) wie in Anspruch 1 definiert, welches umfaßt:
(a) Hydrieren einer Verbindung der Formel

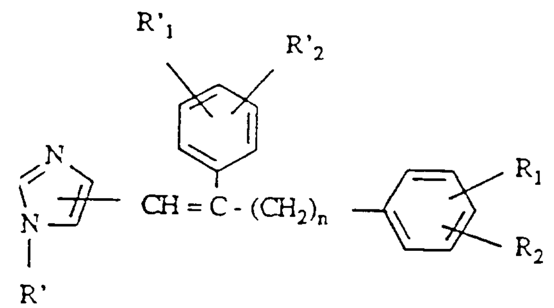

worin R' und n wie in Anspruch 1 definiert sind und $R_1$, $R_2$, $R'_1$ und $R'_2$, die gleich oder verschieden sein können, H, $CH_3$, $C_2H_5$, $C_3H_7$, $OCH_3$, $NO_2$, $NH_2$, CN, $CF_3$, $CHF_2$, $CH_2F$ oder Halogen sind, um eine Verbindung der Formel (I) zu ergeben, die

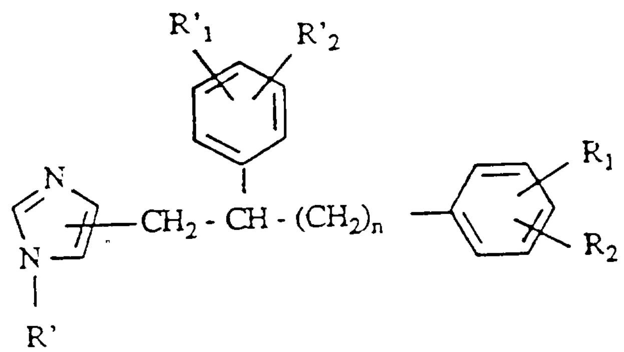

ist, worin R', $R_1$, $R_2$, $R'_1$, $R'_2$ und n wie oben definiert sind, oder
(b) Hydrieren einer Verbindung der Formel

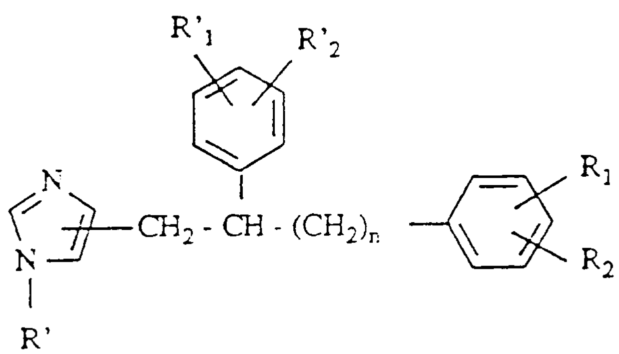

worin $R_1$, $R_2$, $R'_1$, $R'_2$ und n wie oben definiert sind und R' eine substituierte oder unsubstituierte Benzylgruppe ist, um eine Verbindung der Formel (I) zu ergeben, die

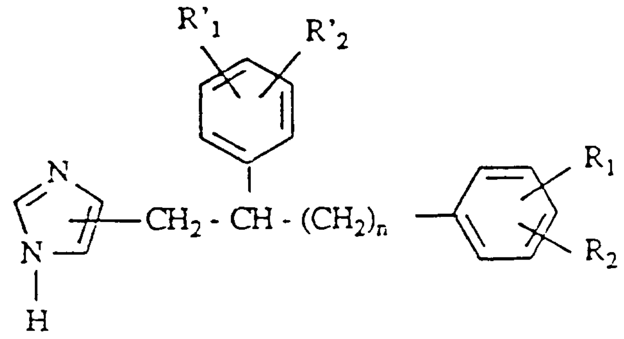

ist, worin $R_1$, $R_2$, $R'_1$, $R'_2$ und n wie oben definiert sind,
und, falls gewünscht, Überführen der resultierenden Verbindung der Formel (I) in ein nicht-toxisches, pharmazeutisch annehmbares Säureadditionssalz derselben.

**12.** Verfahren zur Herstellung eines therapeutisch aktiven substituierten Imidazols der Formel (I) wie in Anspruch 1 definiert, welches umfaßt:
Hydrieren einer Verbindung der Formel

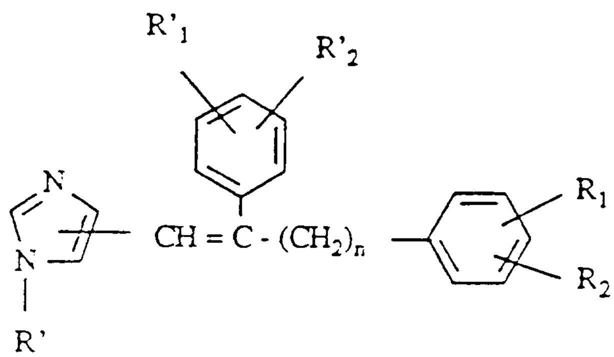

worin R', $R_1$, $R_2$, $R'_1$, $R'_2$ und n wie in Anspruch 11 definiert sind, um eine Verbindung der Formel (I) zu ergeben, die

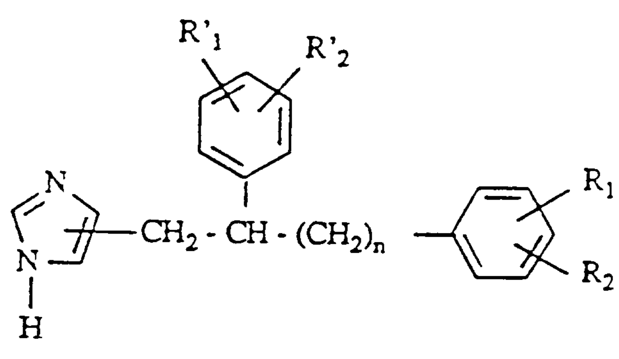

ist, worin $R_1$, $R_2$, $R'_1$, $R'_2$ und n wie in Anspruch 11 definiert sind,
und, falls gewünscht, Überführen der resultierenden Verbindung der Formel (I) in ein nicht-toxisches, pharmazeutisch annehmbares Säureadditionssalz derselben.

**13.** Verfahren zur Herstellung eines therapeutisch aktiven substituierten Imidazols der Formel (I) wie in Anspruch 1 definiert, welches umfaßt:
Umsetzen einer Verbindung der Formel

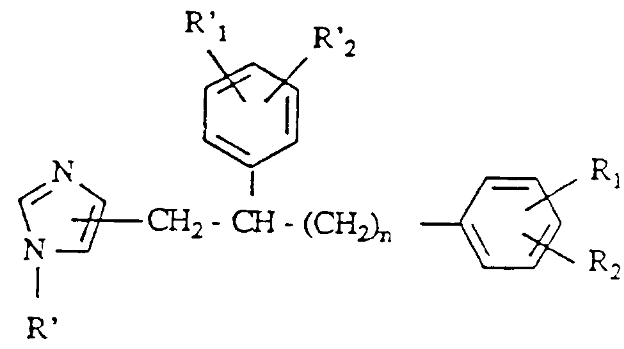

worin R' eine substituierte oder unsubstituierte Benzylgruppe und n 1 bis 4 ist und $R_1$, $R_2$, $R'_1$, $R'_2$, die gleich oder verschieden sein können, H, $CH_3$, $C_2H_5$, $C_3H_7$, $OCH_3$, $NO_2$, $NH_2$, $CF_3$, $CHF_2$, $CH_2F$ oder Halogen sind, durch eine Wasserstoffübertragungsreaktion, um eine Verbindung der Formel (I) zu ergeben, die

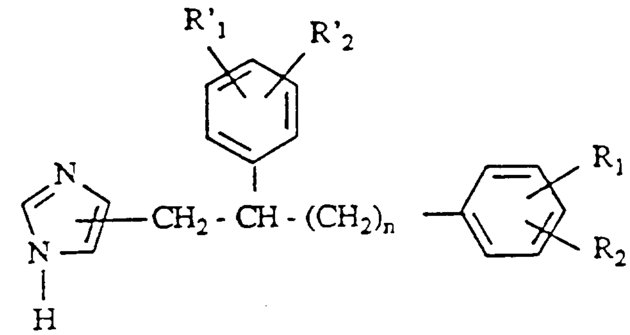

ist, worin $R_1$, $R_2$, $R'_1$, $R'_2$ und n wie oben definiert sind,
und, falls gewünscht, Überführen der resultierenden Verbindung der Formel (I) in ein nicht-toxisches, pharmazeutisch annehmbares Säureadditionssalz derselben.

**14.** Verfahren zur Herstellung eines therapeutisch aktiven substituierten Imidazols der Formel (I) wie in Anspruch 1 definiert, welches umfaßt:
Umsetzen einer Verbindung der Formel

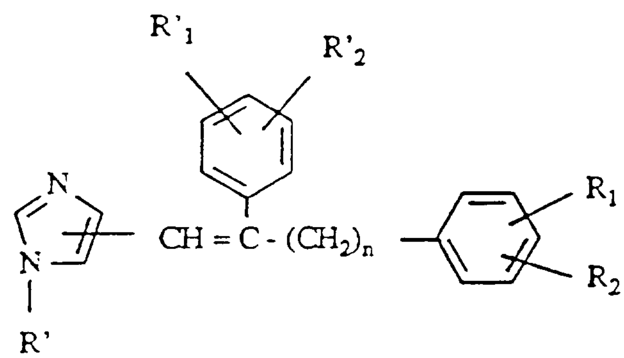

worin R' und n wie in Anspruch 1 definiert sind und $R_1$, $R_2$, $R'_1$ und $R'_2$ wie in Anspruch 13 definiert sind, durch Wasserstoffübertragungsreaktion, um eine Verbindung der Formel (I) zu ergeben, die

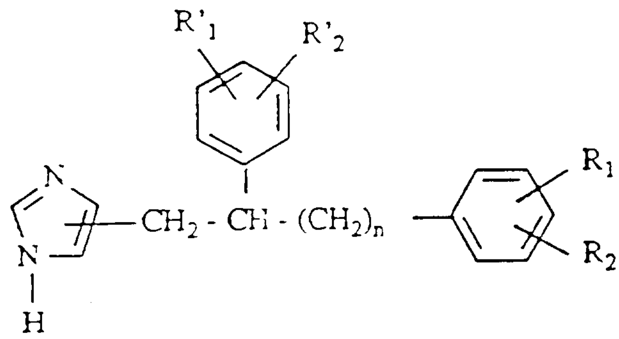

ist, worin $R_1$, $R_2$, $R'_1$ und $R'_2$ wie in Anspruch 13 definiert sind, und, falls gewünscht, Überführen der resultierenden Verbindung der Formel (I) in ein nicht-toxisches, pharmazeutisch annehmbares Säureadditionssalz derselben.

**Revendications**

**Revendications pour les Etats contractants suivants : AT, BE, CH, DE, DK, FR, GB, IT, LI, LU, NL, SE**

**1.** Composé qui est un imidazole substitué de formule :

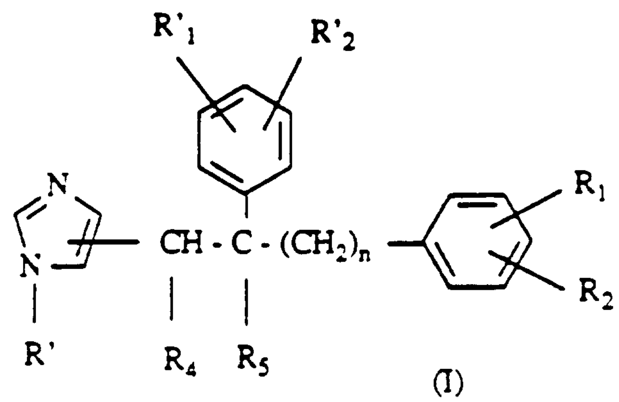

(I)

ou un sel d'addition d'acide non toxique de celui-ci, pharmaceutiquement acceptable, dans laquelle $R_1$, $R_2$, $R'_1$ et $R'_2$ qui peuvent être identiques ou différents, sont des atomes d'hydrogène, des groupes $CH_3$, $C_2H_5$, $C_3H_7$, $OCH_3$, $NO_2$, $NH_2$, CN, $CF_3$, $CHF_2$, $CH_2F$, ou des atomes d'halogène; R' est un atome d'hydrogène ou un groupe :

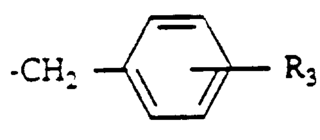

dans lequel $R_3$ représente un atome d'hydrogène, un groupe $CH_3$, ou un atome d'halogène; $R_4$ est un atome d'hydrogène ou un groupe OH, et $R_5$ est un atome d'hydrogène ou un groupe OH, ou bien $R_4$ et $R_5$ ensemble forment une liaison, et n est égal à 1, 2, 3, ou 4.

**2.** Composé selon la revendication 1, dans lequel $R_4$ et $R_5$ sont tous deux des atomes d'hydrogène.

**3.** Composé selon la revendication 1 ou la revendication 2, dans lequel $R_1$, $R_2$, $R'_1$, et $R'_2$ représentent chacun un atome d'hydrogène.

**4.** Composé selon la revendication 1 ou la revendication 2, dans lequel $R_1$ et $R'_1$ sont tous des atomes d'hydrogène, et $R_2$ et $R'_2$, qui sont tels que définis dans la revendication 1, sont tous deux en position para du groupe phényle.

**5.** Composé selon l'une quelconque des revendications 1 à 4, dans lequel $R_2$ et $R'_2$, qui peuvent être identiques ou différents, représentent un groupe CN ou un atome d'halogène.

**6.** Composé selon la revendication 5, dans lequel l'halogène est le fluor.

**7.** Composé selon l'une quelconque des revendications 1 à 6, dans lequel R' est un atome d'hydrogène.

**8.** Composé selon la revendication 1, qui est le 4-(2,4-diphénylbutyl)-1H-imidazole, ou un sel d'addition d'acide de celui-ci, non toxique, pharmaceutiquement acceptable.

**9.** Composé selon la revendication 1, qui est le 4-[2-(4-fluorophényl)-4-phénylbutyl]-1H-imidazole, ou un sel d'addition d'acide de celui-ci, non toxique, pharmaceutiquement acceptable.

**10.** Composé selon la revendication 1, qui est le 4-[2,4-bis(4-fluorophényl)butyl]-1H-imidazole, ou un sel d'addition d'acide de celui-ci, non toxique, pharmaceutiquement acceptable.

**11.** Composé selon la revendication 1, qui est le 4-[2-(4-cyanophényl)-4-(4-fluorophényl)butyl]-1H-imidazole, ou un sel d'addition d'acide de celui-ci, non toxique, pharmaceutiquement acceptable.

**12.** Composition pharmaceutique comprenant un composé selon l'une quelconque des revendications 1 à 11, et un véhicule pharmaceutiquement acceptable.

**13.** Composé selon l'une quelconque des revendications 1 à 11, pour l'utilisation dans une méthode de traitement médical du corps humain ou animal.

**14.** Composé selon la revendication 13, pour l'utilisation en tant qu'inhibiteur de l'aromatase.

**Revendications pour les Etats contractants suivants : ES, GR**

**1.** Procédé pour la préparation d'un imidazole substitué thérapeutiquement actif, de formule :

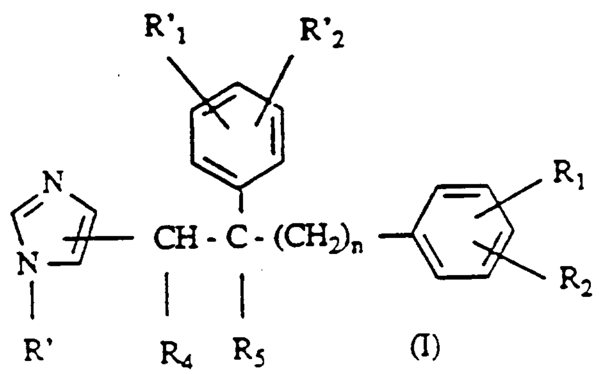

(I)

ou d'un sel d'addition d'acide non toxique de celui-ci, pharmaceutiquement acceptable, dans laquelle $R_1$, $R_2$, $R'_1$ et $R'_2$ qui peuvent être identiques ou différents, sont des atomes d'hydrogène, des groupes $CH_3$, $C_2H_5$, $C_3H_7$, $OCH_3$, $NO_2$, $NH_2$, CN, $CF_3$, $CHF_2$, $CH_2F$, ou des atomes d'halogène; R' est un atome d'hydrogène ou un groupe :

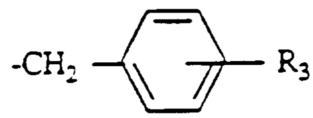

dans lequel $R_3$ représente un atome d'hydrogène, un groupe $CH_3$, ou un atome d'halogène; $R_4$ est un atome d'hydrogène ou un groupe OH, et $R_5$ est un atome d'hydrogène ou un groupe OH, ou bien $R_4$ et $R_5$ ensemble forment une liaison, et n est égal à 1, 2, 3, ou 4,
qui comprend la réaction d'une diphénylcétone de formule :

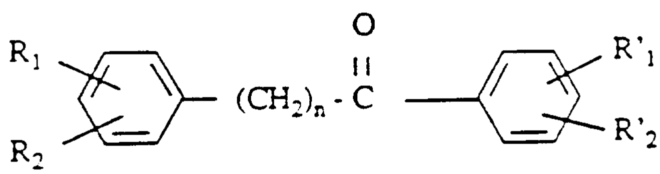

dans laquelle $R_1$, $R_2$, $R'_1$ et $R'_2$ qui peuvent être identiques ou différents, sont des atomes d'hydrogène, des groupes $CH_3$, $C_2H_5$, $C_3H_7$, $OCH_3$, $NH_2$, CN, $CF_3$, $CHF_2$, $CH_2F$, ou des atomes d'halogène, et n est égal à 1, 2, 3 ou 4,
avec un 4(5)-imidazole aldéhyde de formule :

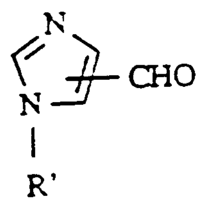

dans laquelle R' est tel que décrit plus haut, en présence d'un réactif au titane de faible valence, pour donner un composé qui est de formule (I) :

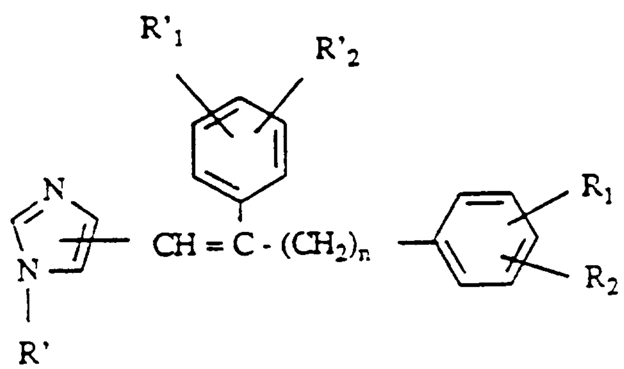

dans laquelle R', $R_1$, $R_2$, $R'_1$, $R'_2$, et n sont tels que définis plus haut, et si désiré, le composé de formule (I) obtenu est transformé en l'un de ses sels d'addition d'acide, non toxique, pharmaceutiquement acceptable.

2. Procédé pour la préparation d'un imidazole substitué thérapeutiquement actif, de formule (I) tel que défini dans la revendication 1, lequel procédé comprend la réaction d'une cétone de formule :

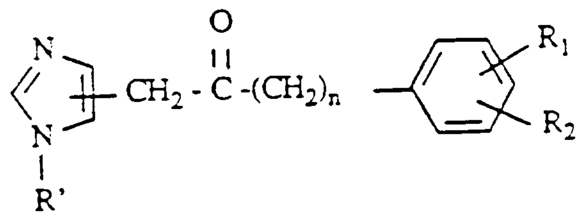

dans laquelle R' et n sont tels que définis dans la revendication 1, et $R_1$, et $R_2$, qui peuvent être identiques ou différents, sont des atomes d'hydrogène, des groupes $CH_3$, $C_2H_5$, $C_3H_7$, $OCH_3$, $NO_2$, $CF_3$, $CHF_2$, $CH_2F$, ou des atomes d'halogène, avec un dérivé halogénure de formule :

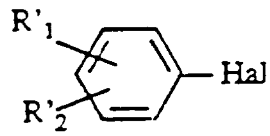

dans laquelle $R'_1$, et $R'_2$, qui peuvent être identiques ou différents sont des atomes d'hydrogène, des groupes $CH_3$, $C_2H_5$, $C_3H_7$, $OCH_3$, $NO_2$, $CF_3$, $CHF_2$, $CH_2F$, ou des atomes d'halogène,
en présence d'un alkyle lithium ou magnésium pour donner un composé de formule :

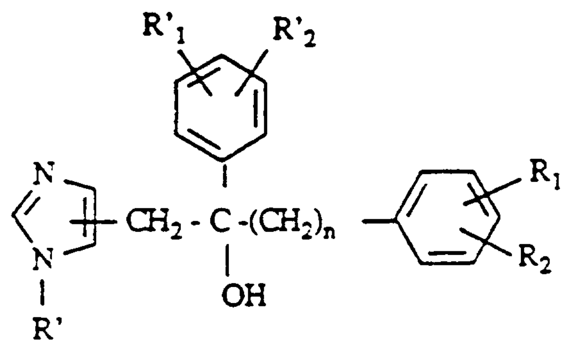

dans laquelle R', $R_1$, $R_2$, $R'_1$, $R'_2$, et n sont tels que définis dans la revendication 1, et si désiré, le composé de formule (I) obtenu est transformé en l'un de ses sels d'addition d'acide, non toxique, pharmaceutiquement acceptable.

3. Procédé pour la préparation d'un imidazole substitué thérapeutiquement actif de formule (I) tel que défini dans la revendication 1, lequel procédé comprend la déshydratation d'un composé de formule :

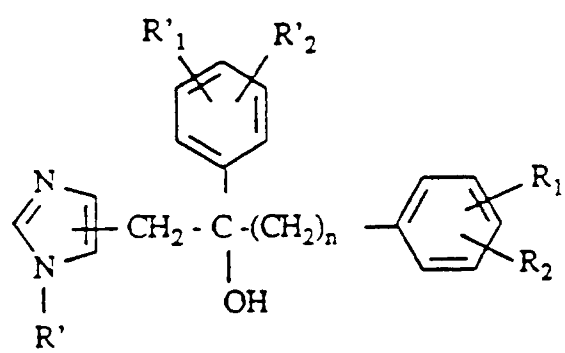

dans laquelle R', $R_1$, $R_2$, $R'_1$, $R'_2$, et n sont tels que définis dans la revendication 2, pour donner un composé qui est de formule (I) :

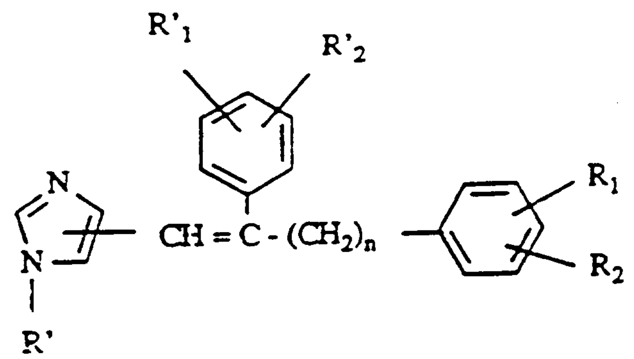

dans laquelle R', $R_1$, $R_2$, $R'_1$, $R'_2$, et n sont tels que définis dans la revendication 2, et si désiré, le composé de formule (I) obtenu est transformé en l'un de ses sels d'addition d'acide, non toxique, pharmaceutiquement acceptable.

**4.** Procédé pour la préparation d'un imidazole substitué thérapeutiquement actif, de formule (I) tel que défini dans la revendication 1, lequel procédé comprend le traitement d'un composé de formule:

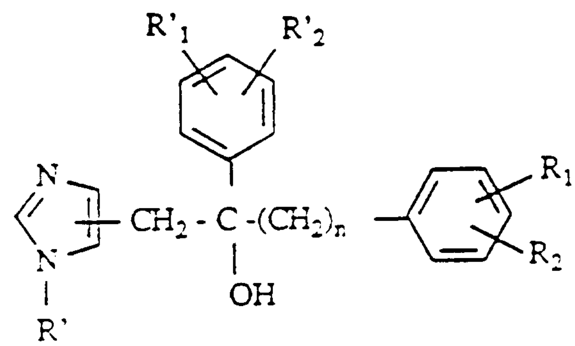

dans laquelle R' est un groupe benzyle protecteur, et $R_1$, $R_2$, $R'_1$, $R'_2$, et n sont tels que définis dans la revendication 2, par une réaction de transfert d'hydrogène, pour donner un composé qui est de formule (I) :

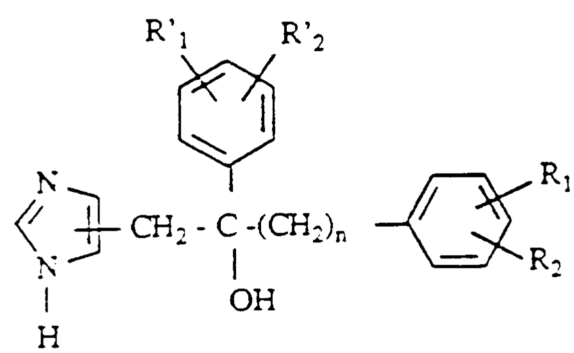

dans laquelle $R_1$, $R_2$, $R'_1$, $R'_2$, et n sont tels que définis dans la revendication 2, et si désiré, le composé de formule (I) obtenu est transformé en l'un de ses sels d'addition d'acide, non toxique, pharmaceutiquement acceptable.

**5.** Procédé pour la préparation d'un imidazole substitué thérapeutiquement actif, de formule (I) tel que défini dans la revendication 1, lequel procédé comprend la réaction d'un diphénylhalogène de formule :

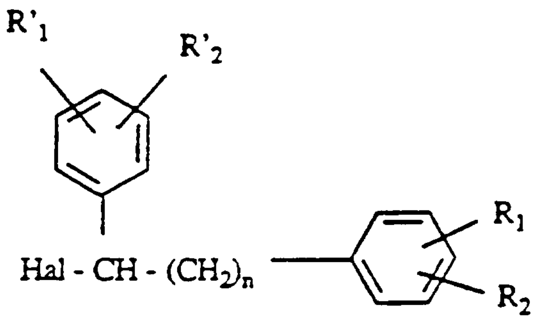

dans laquelle $R_1$, $R_2$, $R'_1$, $R'_2$, et n sont tels que définis dans la revendication 2, et Hal représente un atome d'halogène, avec un 4(5)-imidazole aldéhyde de formule :

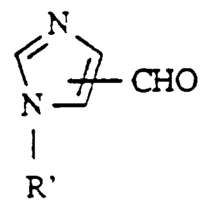

dans laquelle R' est tel que défini dans la revendication 2, en présence de n-butyllithium, pour donner un composé qui est de formule (I) :

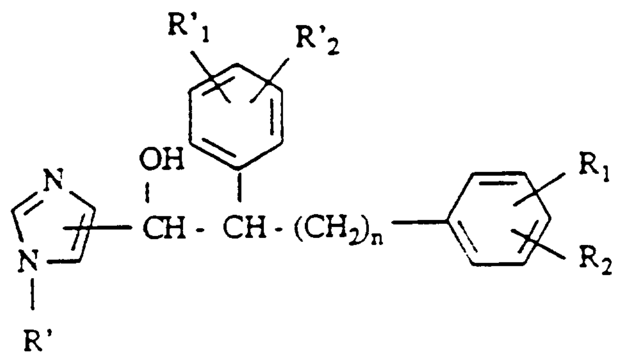

dans laquelle R', $R_1$, $R_2$, $R'_1$, $R'_2$, et n sont tels que définis dans la revendication 2, et si désiré, le composé de formule (I) obtenu est transformé en l'un de ses sels d'addition d'acide, non toxique, pharmaceutiquement acceptable.

**6.** Procédé pour la préparation d'un imidazole substitué thérapeutiquement actif, de formule (I) tel que défini dans la revendication 1, lequel procédé comprend la déshydratation d'un composé de formule :

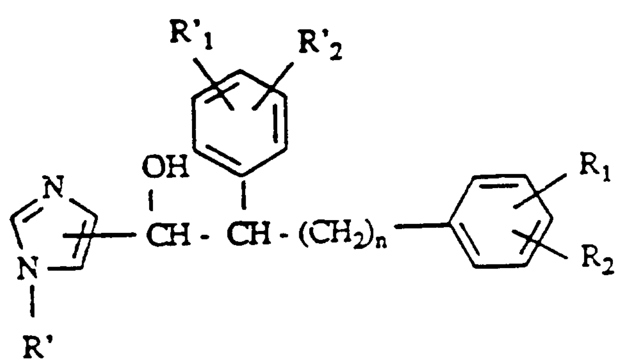

dans laquelle R', $R_1$, $R_2$, $R'_1$, $R'_2$, et n sont tels que définis dans la revendication 2, pour donner un composé qui est de formule (I) :

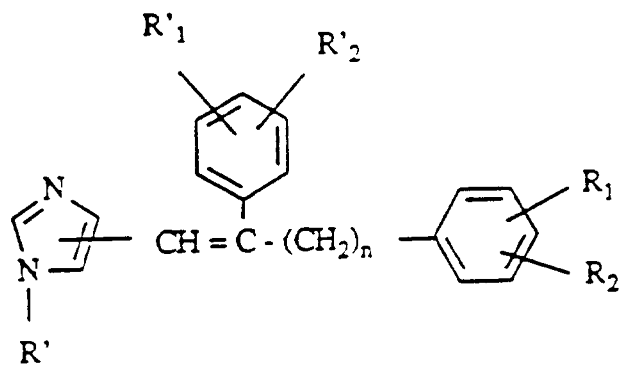

dans laquelle R', $R_1$, $R_2$, $R'_1$, $R'_2$, et n sont tels que définis dans la revendication 2, et si désiré, le composé de formule (I) obtenu est transformé en l'un de ses sels d'addition d'acide, non toxique, pharmaceutiquement acceptable.

**7.** Procédé pour la préparation d'un imidazole substitué thérapeutiquement actif, de formule (I) tel que défini dans la revendication 1, lequel procédé comprend la réaction d'un aldéhyde de formule :

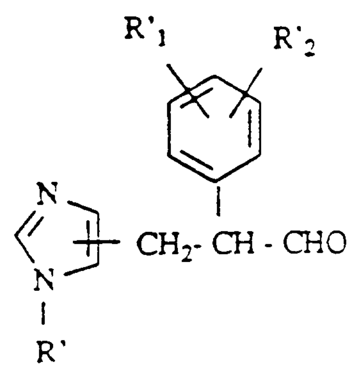

ou d'un amide de formule :

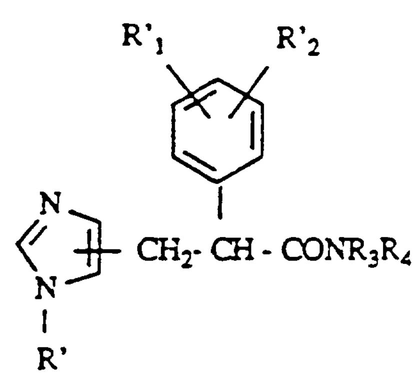

dans laquelle $R'_1$, et $R'_2$, qui peuvent être identiques ou différents, sont des atomes d'hydrogène, des groupes $CH_3$, $C_2H_5$, $C_3H_7$, $OCH_3$, $NO_2$, $CF_3$, $CHF_2$, $CH_2F$, ou des atomes d'halogène; R' est tel que défini dans la revendication 2, et $R_3$ et $R_4$ sont des groupes alkyle inférieur ou N, ou bien $R_3$ et $R_4$ ensemble forment un cycle,
avec un halogénure d'aryle ou d'arylalkyle approprié :

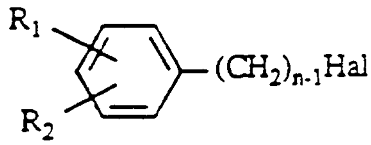

dans lequel $R_1$ et $R_2$ qui peuvent être identiques ou différents, sont des atomes d'hydrogène, des groupes $CH_3$, $C_2H_5$, $C_3H_7$, $OCH_3$, $NO_2$, $NH_2$, CN, $CF_3$, $CHF_2$, $CH_2F$, ou des atomes d'halogène, et n est égal à 1, 2, 3, ou 4,
en présence d'un alkyle lithium ou magnésium pour donner un composé de formule :

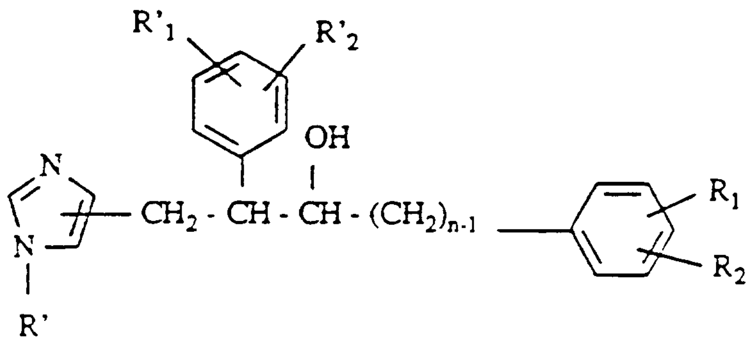

ou

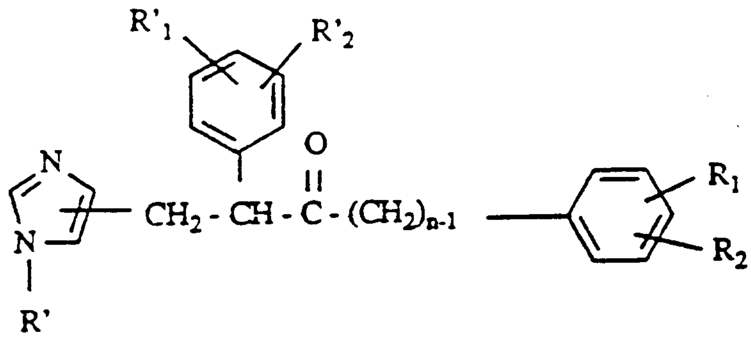

qui est ensuite réduit, déshydraté, et hydrogéné pour donner un composé qui est de formule (I) :

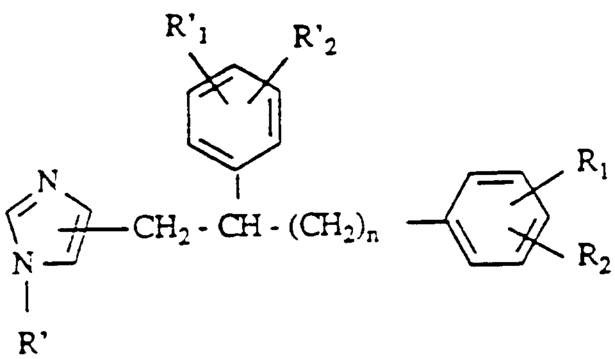

dans laquelle R', et n sont tels que définis plus haut, et $R_1$ $R_2$, $R'_1$, et $R'_2$, qui peuvent être identiques ou différents, sont des atomes d'hydrogène, des groupes $CH_3$, $C_2H_5$, $C_3H_7$, $OCH_3$, $NH_2$, CN, $CF_3$, $CHF_2$, $CH_2F$, ou des atomes d'halogène, et si désiré, le composé de formule (I) obtenu est transformé en l'un de ses sels d'addition d'acide, non toxique, pharmaceutiquement acceptable.

8. Procédé pour la préparation d'un imidazole substitué thérapeutiquement actif de formule (I) tel que défini dans la revendication 1, lequel procédé comprend la réaction d'une cétone de formule :

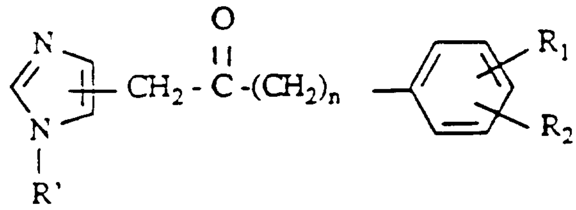

dans laquelle R' représente un groupe benzyle protecteur, et n est égal à 1, 2, 3, ou 4, avec un dérivé halogénure de formule :

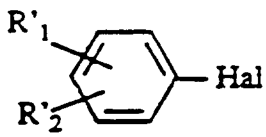

dans laquelle Hal représente un halogène, et un ou plusieurs des substituants $R_1$, $R_2$, $R'_1$ et $R'_2$ représentent un groupe ter-butylaminocarbonyle, ou un groupe oxazoline, et les substituants qui ne sont pas un groupe ter-butylaminocarbonyle, ou un groupe oxazoline, représentent des atomes d'hydrogène, des groupes $CH_3$, $C_2H_5$, $C_3H_7$, $OCH_3$, $CF_3$, $CHF_2$, $CH_2F$, ou des atomes d'halogène, en présence d'un alkyle lithium ou magnésium pour donner un composé de formule :

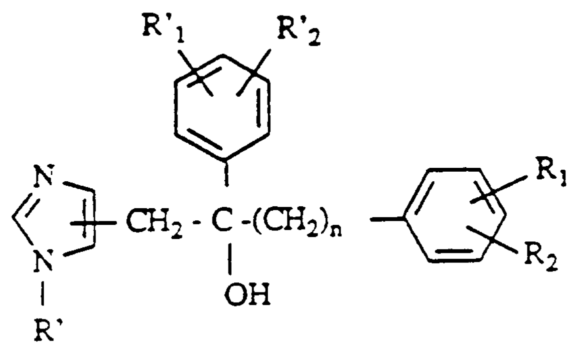

qui est ensuite déshydraté, hydrogéné et mis en réaction avec $SOCl_2$ pour donner un composé qui est de formule (I) :

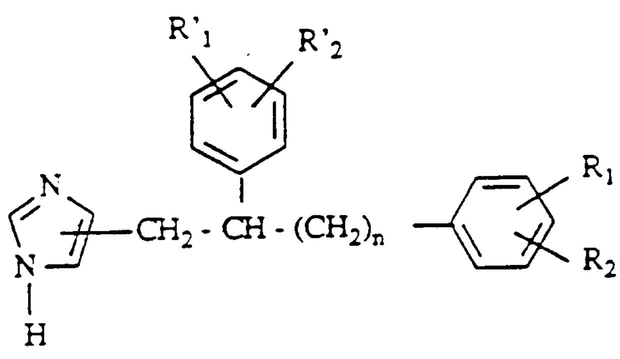

dans laquelle un ou plusieurs des substituants $R_1$, $R_2$, $R'_1$, et $R'_2$, représentent CN, et les substituants qui ne représentent pas CN, sont des atomes d'hydrogène, des groupes $CH_3$, $C_2H_5$, $C_3H_7$, $OCH_3$, $CF_3$,

$CHF_2$, $CH_2F$, ou des atomes d'halogène, et si désiré, le composé de formule (I) obtenu est transformé en l'un de ses sels d'addition d'acide, non toxique, pharmaceutiquement acceptable.

**9.** Procédé pour la préparation d'un imidazole substitué thérapeutiquement actif, de formule (I) tel que défini dans la revendication 1, lequel procédé comprend la réaction d'un composé de formule :

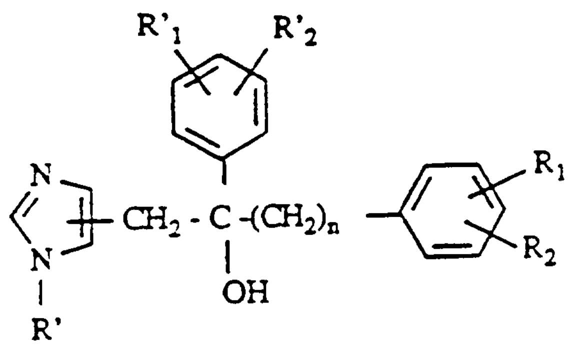

dans laquelle R' représente un groupe benzyle protecteur, et un ou plusieurs des substituants $R_1$, $R_2$, $R'_1$ et $R'_2$ représentent un groupe ter-butylaminocarbonyle, ou un groupe oxazole, et les substituants qui ne sont pas un groupe ter-butylaminocarbonyle, ou un groupe oxazole, sont tels que définis dans la revendication 8,
avec $SOCl_2$, $POCl_3$, ou $PCl_5$, pour donner un composé de formule :

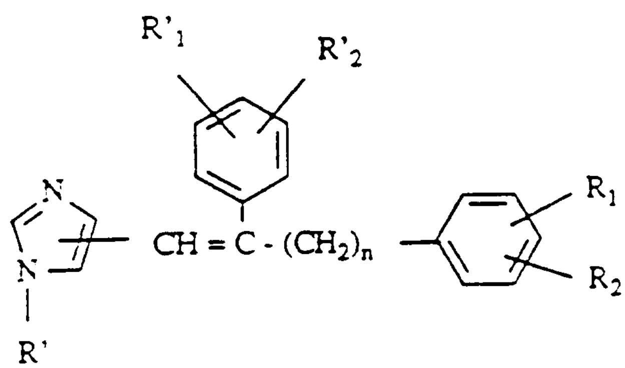

dans laquelle R' représente un groupe benzyle protecteur, un ou plusieurs des substituants $R_1$, $R_2$, $R'_1$ et $R'_2$ représentent un groupe CN, et les substituants qui ne sont pas un groupe CN sont tels que définis dans la revendication 8, et si désiré, le composé de formule (I) obtenu est transformé en l'un de ses sels d'addition d'acide, non toxique, pharmaceutiquement acceptable.

**10.** Procédé pour la préparation d'un imidazole substitué thérapeutiquement actif, de formule (I) tel que défini dans la revendication 1, lequel procédé comprend :

(a) la diazotation d'un composé de formule (I) :

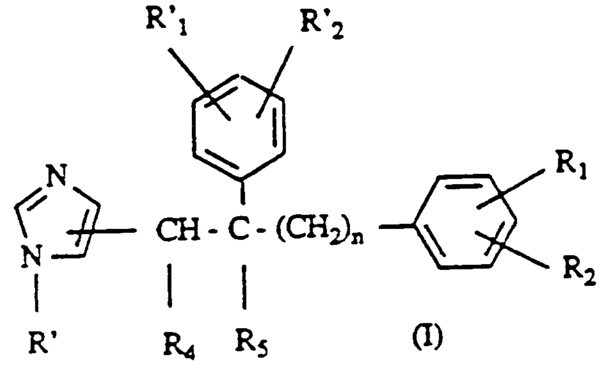

(I)

dans laquelle R', $R_4$, $R_5$, et n sont tels que définis dans la revendication 1, et un ou plusieurs des substituants $R_1$, $R_2$, $R'_1$ et $R'_2$ représentent un groupe $NH_2$, et les substituants qui ne sont pas un groupe $NH_2$ sont tels que définis dans la revendication 1, pour donner un composé de formule (I) dans laquelle un ou plusieurs des substituants $R_1$, $R_2$, $R'_1$ et $R'_2$ représentent un groupe CN, et les substituants qui ne sont pas un groupe CN sont tels que définis dans la revendication 1; ou

(b) l'hydrogénation d'un composé de formule (I);

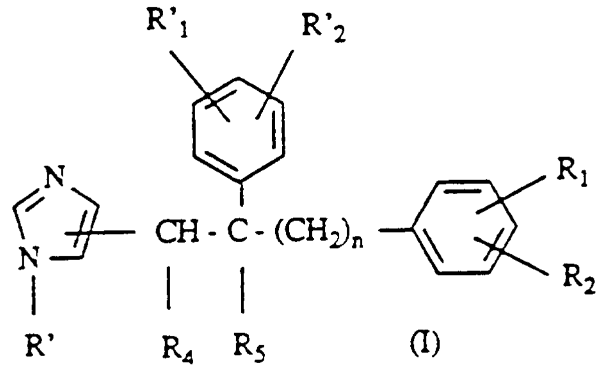

(I)

dans laquelle R', $R_4$, $R_5$, et n sont tels que définis dans la revendication 1, et un ou plusieurs des substituants $R_1$, $R_2$, $R'_1$ et $R'_2$ représentent un groupe $NO_2$, et les substituants qui ne sont pas un groupe $NO_2$ sont tels que définis dans la revendication 1, pour donner un composé de formule (I) dans laquelle R' représente un atome d'hydrogène, $R_4$, $R_5$, et n sont tels que définis plus haut, et un ou plusieurs des substituants $R_1$, $R_2$, $R'_1$ et $R'_2$ représentent un groupe $NH_2$, et les substituants qui ne sont pas un groupe $NH_2$, sont tels que définis dans la revendication 1; ou

(c) la nitration d'un composé de formule (I) :

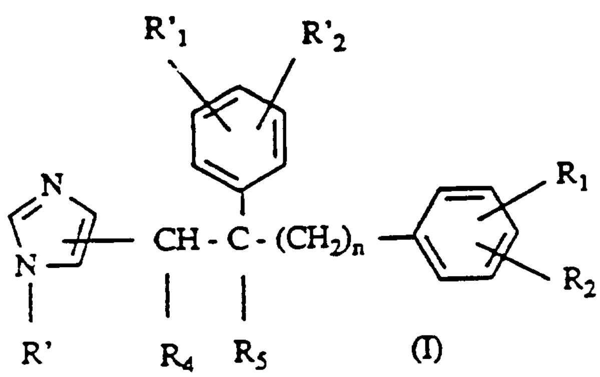

(I)

dans laquelle R', $R_4$, $R_5$, et n sont tels que définis plus haut dans la revendication 1, et un ou plusieurs des substituants $R_1$, $R_2$, $R'_1$ et $R'_2$ représentent un atome d'hydrogène; et les substituants

qui ne sont pas un atome d'hydrogène sont tels que définis dans la revendication 1, pour donner un composé de formule (I) dans laquelle un ou plusieurs des substituants $R_1$, $R_2$, $R'_1$ et $R'_2$ représentent un groupe $NO_2$, et les substituants qui ne sont pas un groupe $NO_2$, sont tels que définis dans la revendication 1;

et si désiré; le composé de formule (I) obtenu est transformé en l'un de ses sels d'addition d'acide, non toxique, pharmaceutiquement acceptable.

**11.** Procédé pour la préparation d'un imidazole substitué thérapeutiquement actif, de formule (I) tel que défini dans la revendication 1, lequel procédé comprend :

(a) l'hydrogénation d'un composé de formule :

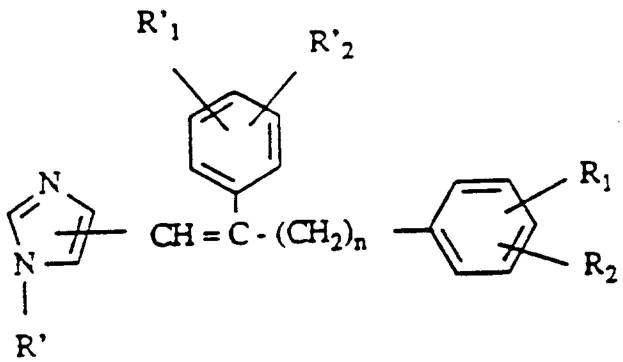

dans laquelle R' et n sont tels que définis dans la revendication 1, et $R_1$, $R_2$, $R'_1$ et $R'_2$ qui peuvent être identiques ou différents, sont des atomes d'hydrogène, des groupes $CH_3$, $C_2H_5$, $C_3H_7$, $OCH_3$, $NO_2$, $NH_2$, CN, $CF_3$, $CHF_2$; $CH_2F$, ou des atomes d'halogène, pour donner un composé qui est de formule (I) :

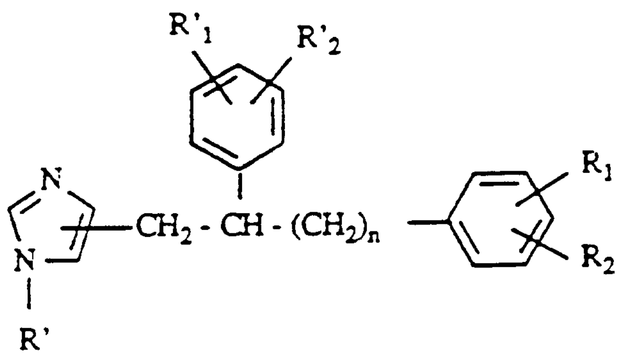

dans laquelle R', $R_1$, $R_2$, $R'_1$ et $R'_2$, et n sont tels que définis plus haut; ou

(b) l'hydrogénation d'un composé de formule :

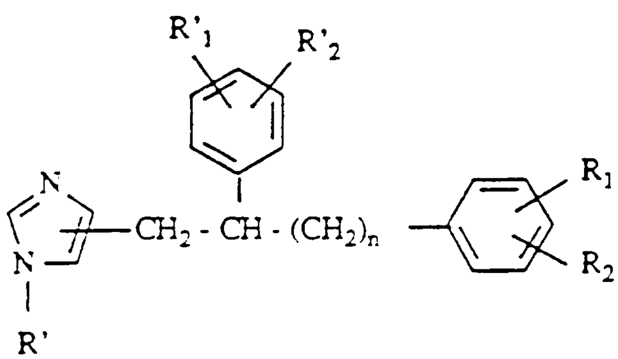

dans laquelle $R_1$, $R_2$, $R'_1$, $R'_2$, et n sont tels que définis plus haut, et R' est un groupe benzyle substitué ou non substitué, pour donner un composé qui est de formule (I) :

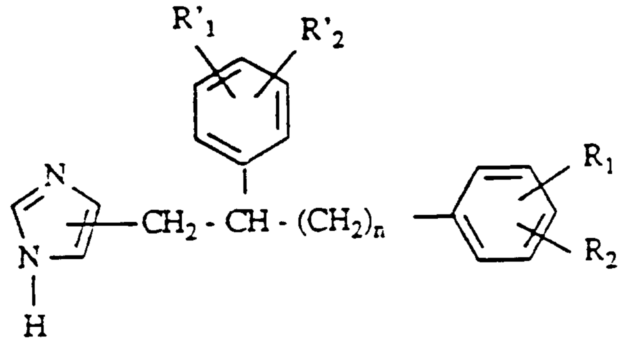

dans laquelle $R_1$, $R_2$, $R'_1$ et $R'_2$, et n sont tels que définis plus haut;
et si désiré; le composé de formule (I) obtenu est transformé en l'un de ses sels d'addition d'acide, non toxique, pharmaceutiquement acceptable.

**12.** Procédé pour la préparation d'un imidazole substitué thérapeutiquement actif, de formule (I) tel que défini dans la revendication 1, lequel procédé comprend l'hydrogénation d'un composé de formule :

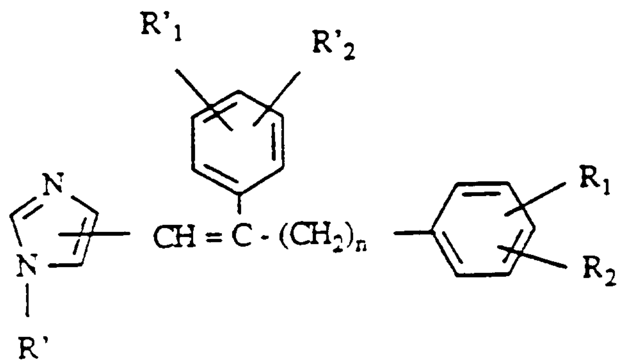

dans laquelle R', $R_1$, $R_2$, $R'_1$, $R'_2$, et n sont tels que définis dans la revendication 11, pour donner un composé qui est de formule (I) :

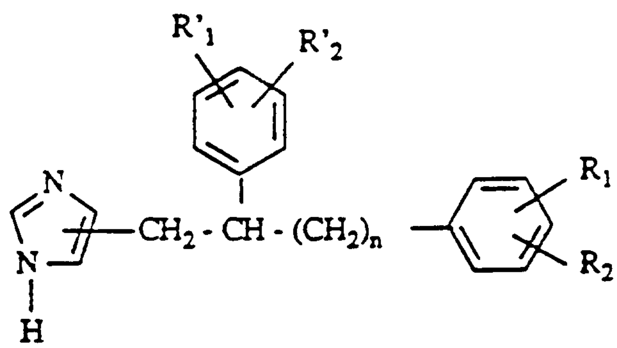

dans laquelle $R_1$, $R_2$, $R'_1$, $R'_2$, et n sont tels que définis dans la revendication 11, et si désiré, le composé de formule (I) obtenu est transformé en l'un de ses sels d'addition d'acide, non toxique, pharmaceutiquement acceptable.

**13.** Procédé pour la préparation d'un imidazole substitué thérapeutiquement actif, de formule (I) tel que défini dans la revendication 1, lequel procédé comprend le traitement d'un composé de formule:

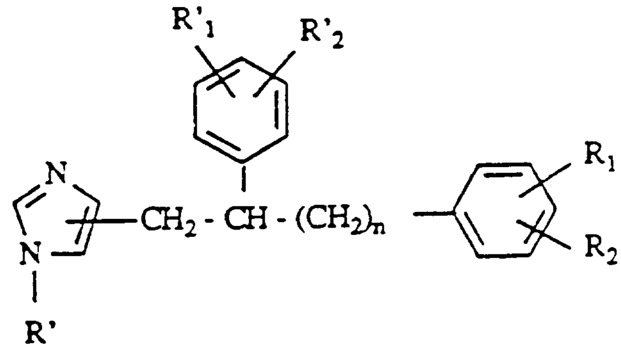

dans laquelle R' est un groupe benzyle substitué ou non substitué, n est égal à 1, 2, 3, ou 4, et $R_1$, $R_2$, $R'_1$ et $R'_2$ qui peuvent être identiques ou différents, sont des atomes d'hydrogène, des groupes $CH_3$, $C_2H_5$, $C_3H_7$, $OCH_3$, $NO_2$, $NH_2$, $CF_3$, $CHF_2$, $CH_2F$, ou des atomes d'halogène, par une réaction de transfert d'hydrogène, pour donner un composé qui est de formule (I) :

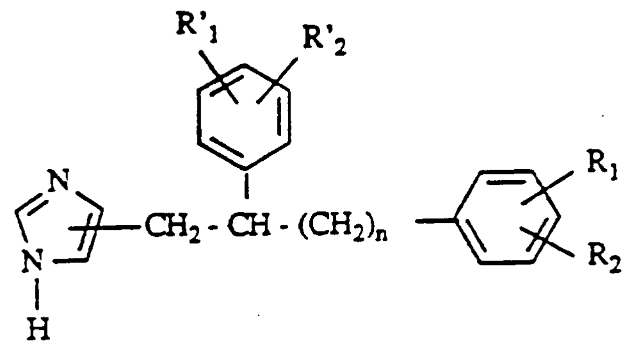

dans laquelle $R_1$, $R_2$, $R'_1$, $R'_2$, et n sont tels que définis plus haut, et si désiré, le composé de formule (I) obtenu est transformé en l'un de ses sels d'addition d'acide, non toxique, pharmaceutiquement acceptable.

**14.** Procédé pour la préparation d'un imidazole substitué thérapeutiquement actif, de formule (I) tel que défini dans la revendication 1, lequel procédé comprend le traitement d'un composé de formule:

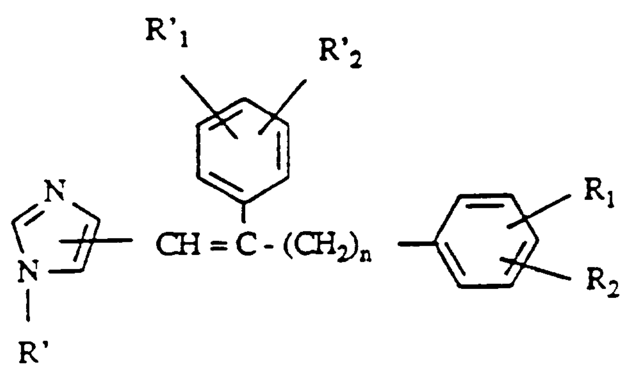

dans laquelle R' et n sont tels que définis dans la revendication 1, et $R_1$, $R_2$, $R'_1$ et $R'_2$ sont tels que définis dans la revendication 13, par une réaction de transfert d'hydrogène, pour donner un composé qui est de formule (I) :

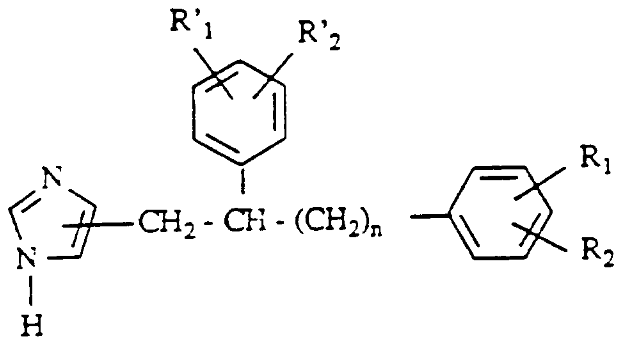

dans laquelle $R_1$, $R_2$, $R'_1$, $R'_2$, et n sont tels que définis dans la revendication 13, et si désiré, le composé de formule (I) obtenu est transformé en l'un de ses sels d'addition d'acide, non toxique, pharmaceutiquement acceptable.